# EUROPEAN PATENT SPECIFICATION

(11) **EP 3 886 813 B1**
(45) Date of publication and mention of the grant of the patent: **22.03.2023**
(21) Application number: 19821392.8
(22) Date of filing: 22.11.2019
(51) Int. Cl.: A61K 9/107, A61K 38/13, A61K 47/10, A61K 47/14, A61K 47/26, A61P 31/20

(54) **PHARMACEUTICAL FORMULATIONS OF CYCLOSPORINE ANALOGS**
PHARMAZEUTISCHE FORMULIERUNGEN VON CYCLOSPORINANALOGA
FORMULATIONS PHARMACEUTIQUES D'ANALOGUES DE CYCLOSPORINE

(30) Priority: 26.11.2018 US 201862771453 P
(43) Date of publication of application: 06.10.2021
(73) Proprietor: Hepion Pharmaceuticals, Inc., Edison NJ 08837 (US)
(72) Inventor: TREPANIER, Daniel, Joseph, Edison, NJ 08837 (US); URE, Daren, Raymond, Edison, NJ 08837 (US); FOSTER, Robert, Thomas, Edison, NJ 08837 (US)
(74) Representative: J A Kemp LLP
(86) International application number: PCT/US2019/062849
(87) International publication number: WO 2020/112562

(56) References cited:
- EP-A1- 1 059 913
- US-A- 5 962 014
- US-A1- 2006 014 788
- US-A1- 2018 296 588

## Description

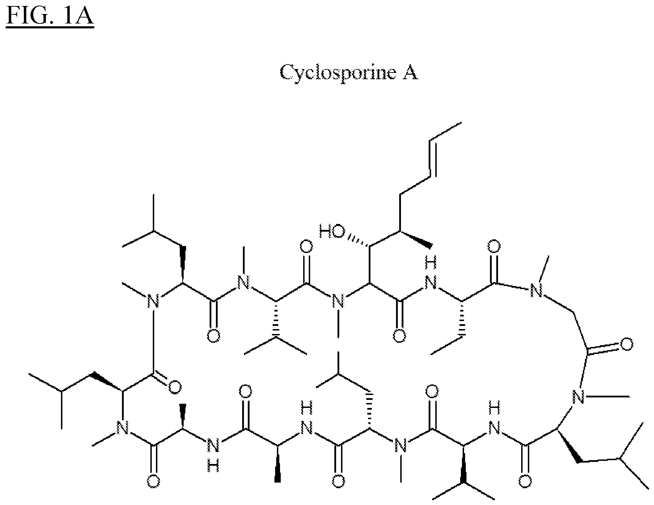

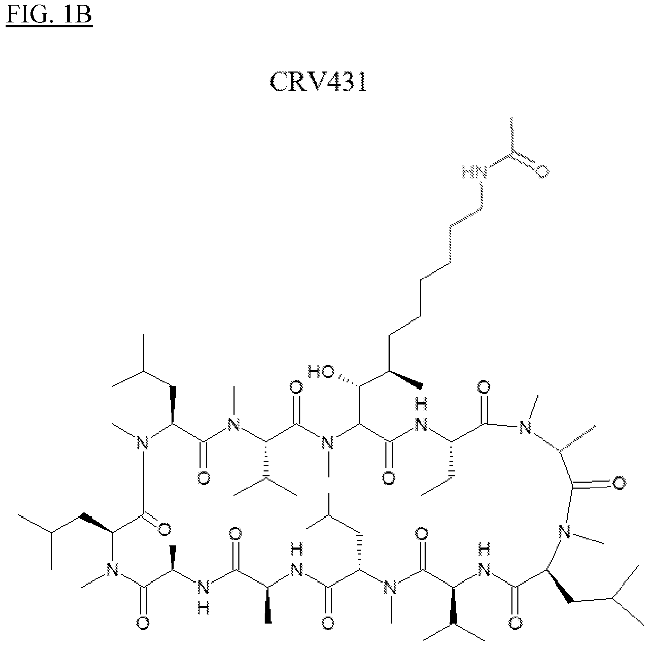

CRV431 is a small molecule cyclophilin inhibitor under clinical development for the treatment of liver diseases including liver fibrosis and hepatocellular carcinoma. In preclinical studies CRV431 has shown anti-viral activity against a number of viruses including hepatitis B, hepatitis C, and HIV and anti-fibrotic activity in the liver in a number of *in vivo* models. CRV431 (shown in FIG. 1B) is a derivative of cyclosporine A (CsA) (shown in FIG. 1A), a neutral cyclic peptide consisting of eleven amino acids, wherein amino acids 1 and 3 have been chemically modified as shown in FIG. 1B.

The present disclosure provides a stable SMEDDS formulation of CRV431 that enables good solubility of CRV431 and enables significant blood exposure in humans following a single oral dose in healthy subjects.

The present invention therefore provides a self-microemulsifying drug delivery system (SMEDDS) comprising CRV431: 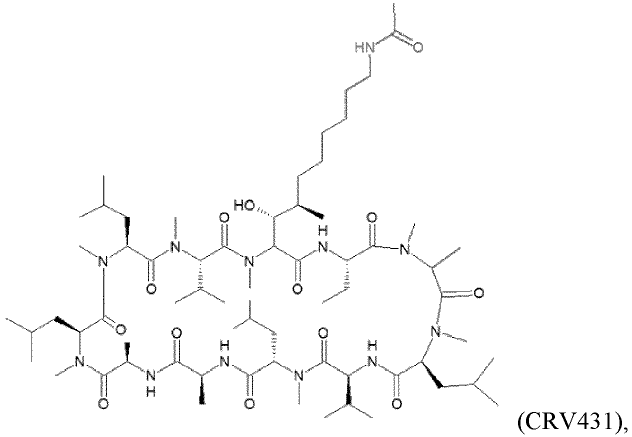 or a pharmaceutically acceptable salt thereof, the SMEDDS further comprising Vitamin E, Maisine^{®} CC, propylene glycol, Transcutol^{®}, ethanol, and Cremophor^{®} RH40 at a weight ratio, respectively, of about (0.75-1.5)/(0.5-2)/(2-5)/(2-5)/(2-2.4)/(4-8).

Cremophor^{®} RH40 is polyoxyl castor oil (also known as polyoxyl 40 hydrogenated castor oil, macrogolglycerol hydroxystearate, and PEG-40 hydrogenated castor oil, such as Kolliphor^{®} RH40). Transcutol^{®} is diethylene glycol monoethyl ether (also known as 2-(2-Ethoxyethoxy)ethanol). Propylene glycol may be abbreviated as PG. Maisine^{®} CC is glyceryl monolinoleate. In some embodiments, the system comprises Vitamin E, Maisine^{®} CC, propylene glycol, Transcutol^{®}, ethanol, and Cremophor^{®} RH40 at a weight ratio of 1/1/5/5/2.4/4 or 1/1.5/2.5/5/2.4/5. In some embodiments, the system comprises CRV431 at a concentration of from about 10 mg/mL to about 90 mg/mL. In some embodiments, the system comprises CRV431 at a concentration of about 90 mg/mL.

The present disclosure also provides a pharmaceutical composition comprising:
(a) CRV431, at a concentration of from about 10 mg/mL to about 90 mg/mL: 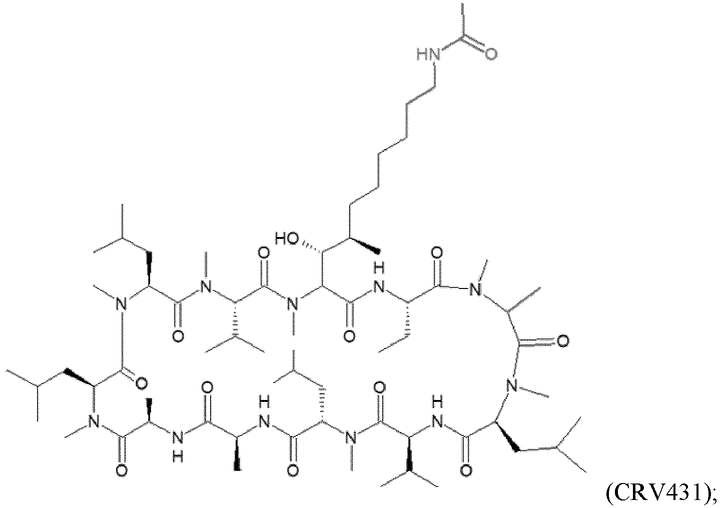
(b) Vitamin E;
(c) Maisine^{®} CC;
(d) propylene glycol;
(e) Transcutol^{®};
(f) ethanol; and
(g) Cremophor^{®} RH40, wherein Vitamin E, Maisine^{®} CC, propylene glycol, Transcutol^{®}, ethanol, and Cremophor^{®} RH40 are at a weight ratio, respectively, of about (0.75-1.5)/(0.5-2)/(2-5)/(2-5)/(2-2.4)/(4-8).

In another aspect, the present disclosure provides a self-microemulsifying drug delivery system for use in the treatment or prevention of a disease in a subject.

In some embodiments, the disease is a disease such as a severe liver disease. In some embodiments, the disease is hepatitis B (HBV), liver fibrosis, or hepatocellular carcinoma. In some embodiments, the disease is hepatitis B (HBV). In some embodiments, the disease is hepatocellular carcinoma.

In some embodiments, an area under curve (AUC) of a plot of a concentration of CRV431 in a blood of the subject over time is from about 5000 ng.hr/ml to about 150000 ng.hr/ml. In some embodiments, a maximum concentration (Cₘₐₓ) of CRV431 in a blood of the subject is from about 1500 ng/ml to about 2500 ng/ml. In some embodiments, a time (Tₘₐₓ) to reach a maximum concentration of CRV431 in a blood of the subject is from about 0.5 hour to about 8 hours. In some embodiments, an elimination half-life (T_{1/2}) of CRV431 in a blood of the subject is from about 10 hours to about 200 hours. In some embodiments, a concentration of CRV431 in a liver of the subject relative to a concentration of CRV431 in a blood of the subject is from about 1 to about 20.

In some embodiments, the therapeutically effective amount of the SMEDDS is from about 0.5 mg/kg to about 5 mg/kg.

In some embodiments, thereby a symptom of the disease in the subject is alleviated and/or a severity of the disease in the subject decreases. In some embodiments, thereby a function of a liver of the subject is improved. In some embodiments, thereby a load of a virus causing the disease decreases.

In some embodiments, the pharmaceutical composition or the SMEDDS is stable at room temperature. In some embodiments, the pharmaceutical composition or the SMEDDS is stable for from at least about 25 days to at least about 200 days. In some embodiments, particles formed by the pharmaceutical composition or the SMEDDS dispersed in an aqueous solution is from about 15 nm to about 40 nm.

The foregoing and other aspects of the present disclosure will now be described in more detail with respect to the description and methodologies provided herein. It should be appreciated that the invention can be embodied in different forms and should not be construed as limited to the embodiments set forth herein. Rather, these embodiments are provided so that this disclosure will be thorough and complete, and will fully convey the scope of the invention to those skilled in the art.

### BRIEF DESCRIPTION OF THE DRAWINGS

FIG. 1A shows the chemical structure of cyclosporine A.
FIG. 1B shows the chemical structure of CRV431.
FIG. 2 is a pseudo-ternary phase diagram showing oil/water microemulsion region for ratios of Cremophor^{®} RH40, Maisine^{®} CC, and co-solvents.
FIG. 3 is a plot of the stability of CRV431 Series 1 SMEDDS by LC-ESI-MS.
FIG. 4 is a plot of the Series 2 SMEDDS stability compared with Series 1.
FIGS. 5 and 6 are plots of CRV431 SMEDDS aqueous dispersion particle diameter measurements.
FIG. 7A is a plot of the pharmacokinetics of CRV431 in humans on the linear scale.
FIG. 7B is a plot of the pharmacokinetics of CRV431 in humans on the log scale for the 75 mg dose.
FIG. 7C is a plot of the pharmacokinetics of CRV431 in humans on the log scale for the 225 mg dose.
FIG. 8 is a plot of whole blood and liver levels of CRV431 in Sprague Dawley rats.

### DETAILED DESCRIPTION

### General Definitions

The terminology used in the disclosure is for the purpose of describing particular embodiments only and is not intended to be limiting of the invention. As used in the description of the embodiments of the invention and the appended claims, the singular forms "a", "an" and "the" are intended to include the plural forms as well, unless the context clearly indicates otherwise. Also, as used herein, "and/or" refers to and encompasses any and all possible combinations of one or more of the associated listed items. Furthermore, the term "about," as used herein when referring to a measurable value such as an amount of a compound, dose, time, temperature, and the like, is meant to encompass variations of 20%, 10%, 5%, 1%, 0.5%, or even 0.1% of the specified amount.

It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Unless otherwise defined, all terms, including technical and scientific terms used in the description, have the same meaning as commonly understood by one of ordinary skill in the art to which this invention belongs.

The term "consists essentially of" (and grammatical variants), as applied to the compositions of this disclosure, means the composition can contain additional components as long as the additional components do not materially alter the composition. The term "materially altered," as applied to a composition, refers to an increase or decrease in the therapeutic effectiveness of the composition of at least about 20% or more as compared to the effectiveness of a composition consisting of the recited components.

As used herein, "Hepatitis B virus" (or "HBV") as used herein is intended to include all subtypes (adw, adr, ayw, and ayr) and or genotypes (A, B, C, D, E, F, G, and H) thereof. The SMEDDS disclosed herein can be used to treat HBV.

As used herein, "a therapeutically effective amount" refers to an amount that will provide some alleviation, mitigation, and/or decrease in at least one clinical symptom in the subject. Those skilled in the art will appreciate that the therapeutic effects need not be complete or curative, as long as some benefit is provided to the subject.

"Treating", includes any effect, *e.g*., lessening, reducing, modulating, or eliminating, that results in the improvement of the condition, disease, disorder, etc. "Treating" or "treatment" of a disease state includes: (1) inhibiting the disease state, *i.e*., arresting the development of the disease state or its clinical symptoms; (2) relieving the disease state, *i.e.,* causing temporary or permanent regression of the disease state or its clinical symptoms; or (3) reducing or lessening the symptoms of the disease state.

In some embodiments, treatment may be administered after one or more symptoms have developed. In other embodiments, treatment may be administered in the absence of symptoms. Treatment may also be continued after symptoms have resolved.

As used herein, the terms "prevention," "prevent," and "preventing" refer to causing the clinical symptoms of the disease state not to develop in a subject that may be exposed to or predisposed to the disease state, but does not yet experience or display symptoms of the disease state. In some embodiments, prevention may be administered in the absence of symptoms. For example, prevention may be administered to a susceptible individual prior to the onset of symptoms (e.g., in light of a history of symptoms and/or in light of genetic or other susceptibility factors). Prevention may also be continued after symptoms have resolved, for example to delay their recurrence.

"SMEDDS" refers to a self-microemulsifying drug delivery system.

### CRV431 Solubility

The present disclosure provides a self-microemulsifying drug delivery system (SMEDDS) formulation for the oral delivery of a derivative of cyclosporine A (CsA), CRV431, a non-immunosuppressive analogue of cyclosporine A. In some embodiments, relative to cyclosporine A, CRV431 can be poorly soluble in lipid solvents and thusly presents a challenge for the development of a formulation of sufficient oral bioavailability for clinical use. Accordingly, the present disclosure provides a SMEDDS system for delivery of CRV431 to patients (e.g., humans).

As set forth herein, the solubility of CRV431, a cyclosporine A derivative, was determined in a range of commonly used surfactants, oils and co-solvents. A pseudo-ternary phase diagram was constructed from the most soluble excipients and prototype formulations, and SERIES 1 and SERIES 2 were developed. The pharmacokinetics, following single oral doses of 1 and 3 mg/kg of CRV431 SMEDDS, was studied in healthy human volunteers using liquid chromatography-electrospray ionization-mass spectrometry (LC-ESI-MS).

The maximum drug load for the SERIES 1 formulations was less than 40 mg/ml. Adjustment of the excipient ratios allowed for the development of SERIES 2 formulations, which had higher drug loading capacity and stability for CRV431 compared to SERIES 1. Further adjustment allowed for the development of a SMEDDS formulation containing up to 90 mg/ml CRV431 and which generated a microemulsion mean particle size of 25 nm when dispersed into aqueous media. The pharmacokinetics of the CRV431 SMEDDS disclosed herein displayed excellent total body exposure and dose-proportional effects in humans, and high drug levels in the liver of rats.

In some embodiments, the SMEDDS formulation disclosed herein can be used for effective clinical development of CRV431. For example, formulations disclosed herein can be targeted to the treatment of liver diseases including hepatitis B (HBV), liver fibrosis, and hepatocellular carcinoma.

The aqueous solubility of CRV431 was found to be approximately 4-fold greater than CsA (115 vs 30 µg/ml), and this was also reflected in the calculated polar surface area (308 vs 273 angstroms). Without wishing to be bound by theory, while the increase in water solubility might be expected to result in lower plasma protein binding (higher free faction), CRV431 is still considered sparingly soluble in water and therefore without wishing to be bound by theory would not be expected to have any appreciable clinical utility if formulated in a solid tablet. The calculated Log P for CRV431 (3.98) is still very high and, consequently, its solubility in lipid would be expected to be similar to cyclosporine A. The actual solubility of CRV431 in various lipid surfactants and oils is, however, far less than that of cyclosporine A (Table 3). Without wishing to be bound by theory, the intrinsic solubility of CRV431 in any given SMEDDS formulation would be expected to be significantly less than that of CsA, leading to a lower loading capacity. Without wishing to be bound by theory, for a microemulsion to form a SMEDDS formulation must, at least, contain an oil/surfactant mixture. Into this mixture various co-solvents can be added to enhance drug solubility.

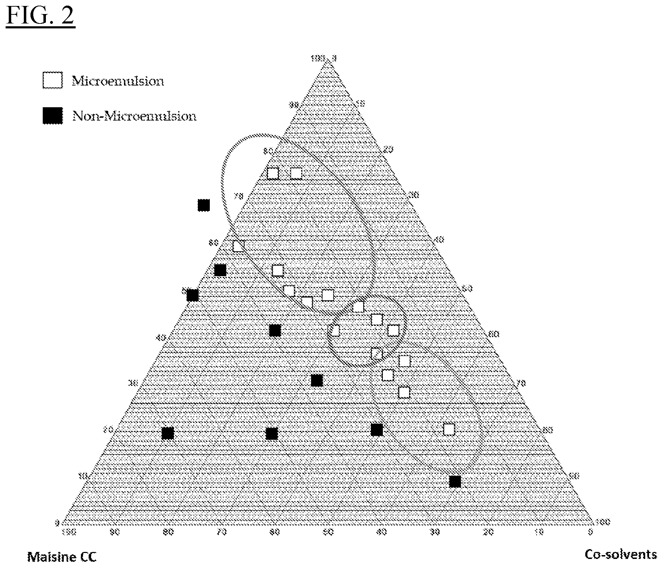

In some embodiments, the surfactant with the highest solubility for CRV431 included Cremophor^{®} RH40, Lauroglycol^{™} 90 and Capryol^{™} 90. Both Lauroglycol^{™} 90 and Capryol^{™} 90 are water-insoluble surfactants and without wising to be bound by theory would not be expected to be useful in formation of oil-in-water microemulsions. Mixtures of either Lauroglycol^{™} 90 or Capryol^{™} 90 with oil and co-solvent excipients were observed to form oil-droplet in water rather than miscible dispersions (data not shown). Therefore, owing to the CRV431 solubility in Cremophor^{®} RH40, Cremophor^{®} RH40 was chosen as the surfactant. Based on the solubility of CRV431 in the oils and co-solvents tested, the excipients: Vitamin E; Maisine^{®} CC; ethanol; Transcutol^{®}; propylene glycol, along with Cremophor^{®} RH40 were chosen for SMEDDS prototype development. A pseudo-ternary phase diagram was constructed by plotting formulation percentages of Maisine^{®} CC (oil) vs Cremophor^{®} RH40 (surfactant) vs. co-solvent mixtures in the absence of drug (FIG. 2). These studies indicated that Cremophor^{®} RH40/Maisine^{®} CC combination ratios less than 3 did not form microemulsions (white opaque by visual inspection). The phase boundary for microemulsion formation (clear in water dispersion) is clearly demarcated and represents approximately 40% of the total area of the phase diagram.

Based on the pseudo-ternary phase diagram a prototype SMEDDS formulation (Table 4: formulation #1) was prepared containing Cremophor^{®} RH40/Maisine^{®} CC/PG/Ethanol/Vitamin E in the ratio of 9/3/3/2/1 and containing 50 mg/ml CRV431. The SMEDDS formulation remained clear for several days after which transparent crystals became apparent and adhered to the bottom of the glass vessel. A series of SMEDDS were developed (SERIES 1, Table 5) wherein the proportion of Transcutol^{®} co-solvent was increased in an attempt to encourage further solubility and decreased crystal formation. All SERIES 1 formulations formed microemulsions when dispersed in water; however, they were also unstable at room temperature. In general, without wishing to be bound by theory, as the level of Transcutol^{®} increased there was an increase in formulation instability. This is likely less to do with the increase in Transcutol^{®} and more to do with the reduction of the other co-solvents in which CRV431 is also moderately soluble. Based on the Series 1 results, Series 2 formulations were produced to explore formulations with greater stability.

Without wishing to be bound by theory, since the pseudo-ternary diagram (FIG. 2) indicates that a microemulsion zone also exists at lower surfactant and oil ratios, the rationale behind the development of SERIES 2 CRV431 SMEDDS formulations was to increase the excipient co-solvents in which CRV431 is most soluble (vitamin E, propylene glycol, Transcutol^{®} and ethanol) and minimize the surfactant and oil components in which CRV431 is least soluble, while maintaining the ability to form a clear microemulsion upon aqueous dispersion.

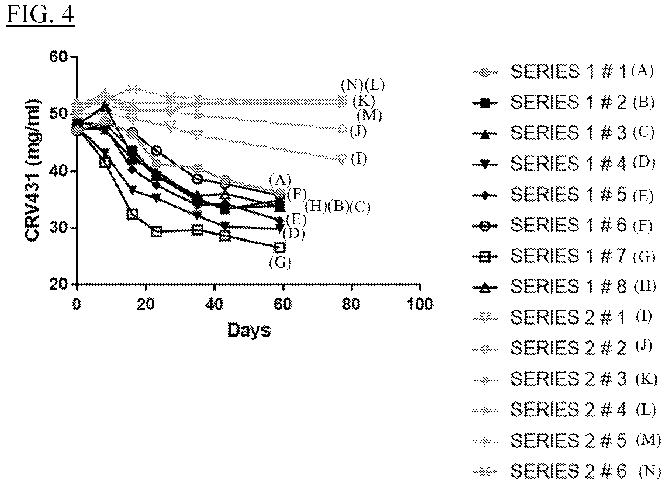

All SERIES 2 SMEDDS have much extended stability (FIG. 4) relative to the original prototype formulation (9/3/3/2/1). Several of the SMEDDS had no observable loss of drug after 77 days storage at room temperature and are, therefore, considered stable. Without wising to be bound by theory, the increased stability is rationalized as resulting from the increased solubility of CRV431 in these SMEDDS such that CRV431 is not at its limit of solubility as was the case for the SERIES 1 SMEDDS. Indeed, SMEDDS 1/1/5/5/2/4 and 1/1.5/2.5/5/2.4/5 (Vitamin E/ Maisine^{®} CC / propylene glycol / Transcutol^{®} / ethanol / Cremophor^{®} RH40 (w/w/w/w/w/w) were also easily prepared at 75 mg/ml. Since the formulation development objective was to increase the CRV431 drug load, the weight ratio of ethanol was increased from 2 to 2.4. In some embodiments, the clinical SMEDDS formulation will be manufactured and delivered to patients as a softgel capsule, therefore further enhancement of ethanol was not considered since this amount of ethanol would be nearing softgel compatibility limits.

The CRV431 SMEDDS (1/1/5/5/2.4/4) was found to have a solubility limit of 90-100 mg/ml and was considered stable. When the 1/1/5/5/2.4/4 and 1/1.5/2.5/5/2.4/5 (Vitamin E/Maisine^{®} CC/Propylene Glycol/Transcutol^{®}/Ethanol/Cremophor^{®} RH40 (w/w/w/w/w/w)) CRV431 SMEDDS were dispersed in aqueous media, the particle size was measured to be approximately 25 nm, which is consistent with the reported particle size (30 nm) of the Neoral^{®} SMEDDS.

The pharmacokinetics of CRV431 SMEDDS (1/1/5/5/2.4/4) was studied in human healthy subjects at 1 and 3 mg/kg. Excellent exposures and dose proportionality were observed. Relative to reported cyclosporine A (2.5 mg/kg) exposures in healthy human subjects, CRV431 Cmax was slightly greater than Neoral^{®}, and overall exposure (AUC) was approximately 14 times greater than Neoral^{®}, which is an oral formulation of cyclosporine A that immediately forms a microemulsion in an aqueous environment and includes Cremophor^{®} RH40 as an inactive ingredient. Without wishing to be bound by theory, this appears to be predominantly due to the large difference in half-life of CRV431, approximately 100 hours, relative to cyclosporine A, which is reported to range from 6.3 hours in healthy subjects to 20.4 hours in severe liver disease.

In rat repeat-dose studies, CRV431 levels in the liver (10 µg/g tissue) were at least 6.5-fold greater than in whole blood.

### SMEDDS of the Disclosure

The relative weight ratio of the different components in the SMEDDS of the present disclosure can vary. The SMEDDS of the present disclosure comprises Vitamin E at a weight ratio of between about 0.75 and about 1.5 (e.g., about 0.75, about 1, about 1.25, about 1.5) relative to the non-CRV431 components in the SMEDDS. In particular, the SEMDDS comprises Vitamin E at a weight ratio of between about 0.75 and about 1.5 (e.g., about 0.75, about 1, about 1.25, about 1.5) relative to each of the Maisine^{®} CC, propylene glycol, Transcutol^{®}, ethanol, and Cremophor^{®} RH40 in the SMEDDS. In some embodiments, the SMEDDS can comprise Vitamin E at a weight ratio of, of about, of at least, or of at most, 0.75, 0.80, 0.85, 0.90, 0.95, 1.00, 1.05, 1.10, 1.15, 1.20, 1.25, 1.30, 1.35, 1.40, 1.45, 1.50, or a number or a range between any two of these values, relative to each of the other non-CRV431 components (including, but not limited to, Maisine^{®} CC, propylene glycol, Transcutol^{®}, ethanol, and Cremophor^{®} RH40) in the SMEDDS.

The SMEDDS of the present disclosure comprises Maisine^{®} CC at a weight ratio of between about 0.5 and about 2 (e.g., about 0.5, about 0.75, about 1, about 1.25, about 1.5, about 1.75, about 2) relative to the non-CRV431 components in the SMEDDS. In particular, the SEMDDS comprises Maisine^{®} CC at a weight ratio of between about 0.75 and about 1.5 (e.g., about 0.75, about 1, about 1.25, about 1.5) relative to each of the Vitamin E, propylene glycol, Transcutol^{®}, ethanol, and Cremophor^{®} RH40 in the SMEDDS. In some embodiments, the SMEDDS can comprise Maisine^{®} CC at a weight ratio of, of about, of at least, or of at most, 0.75, 0.8, 0.85, 0.9, 0.95, 1, 1.05, 1.1, 1.15, 1.2, 1.25, 1.3, 1.35, 1.4, 1.45, 1.5, 1.55, 1.6, 1.65, 1.7, 1.75, 1.8, 1.85, 1.9, 1.95, 2, or a number or a range between any two of these values, relative to each of the other non-CRV431 components (including, but not limited to, Vitamin E, propylene glycol, Transcutol^{®}, ethanol, and Cremophor^{®} RH40) in the SMEDDS.

The SMEDDS of the present disclosure comprises propylene glycol at a weight ratio of between about 2 and about 5 (e.g., about 2, about 2.25, about 2.5, about 2.75, about 3, about 3.25, about 3.5, about 3.75, about 4, about 4.25, about 4.5, about 4.75, about 5) relative to the non-CRV431 components in the SMEDDS. In particular, the SEMDDS comprises propylene glycol at a weight ratio of between about 2 and about 5 relative to each of the Vitamin E, Maisine^{®} CC, Transcutol^{®}, ethanol, and Cremophor^{®} RH40 in the SMEDDS. In some embodiments, the SMEDDS can comprise propylene glycol at a weight ratio of, of about, of at least, or of at most, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, or a number or a range between any two of these values, relative to each of the other non-CRV431 components (including, but not limited to, Vitamin E, Maisine^{®} CC, Transcutol^{®}, ethanol, and Cremophor^{®} RH40) in the SMEDDS.

The SMEDDS of the present disclosure comprises Transcutol^{®} at a weight ratio of between about 2 and about 5 (e.g., about 2, about 2.25, about 2.5, about 2.75, about 3, about 3.25, about 3.5, about 3.75, about 4, about 4.25, about 4.5, about 4.75, about 5) relative to the non-CRV431 components in the SMEDDS. In particular, the SEMDDS comprises Transcutol^{®} at a weight ratio of between about 2 and about 5 relative to each of the Vitamin E, Maisine^{®} CC, propylene glycol, ethanol, and Cremophor^{®} RH40 in the SMEDDS. In some embodiments, the SMEDDS can comprise Transcutol^{®} at a weight ratio of, of about, of at least, or of at most, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, or a number or a range between any two of these values, relative to each of the other non-CRV431 components (including, but not limited to, Vitamin E, Maisine^{®} CC, propylene glycol, ethanol, and Cremophor^{®} RH40) in the SMEDDS.

The SMEDDS of the present disclosure comprises ethanol at a weight ratio of between about 2 and about 2.4 (e.g., about 2; about 2.25; about 2.4) relative to the non-CRV431 components in the SMEDDS. In particular, the SEMDDS comprises ethanol at a weight ratio of between about 2 and about 2.4 relative to each of the Vitamin E, Maisine^{®} CC, propylene glycol, Transcutol^{®}, and Cremophor^{®} RH40 in the SMEDDS. In some embodiments, the SMEDDS can comprise ethanol at a weight ratio of, of about, of at least, or of at most, 2.1, 2.11, 2.12, 2.13, 2.14, 2.15, 2.16, 2.17, 2.18, 2.19, 2.2, 2.21, 2.22, 2.23, 2.24, 2.25, 2.26, 2.27, 2.28, 2.29, 2.3, 2.31, 2.32, 2.33, 2.34, 2.35, 2.36, 2.37, 2.38, 2.39, 2.4, or a number or a range between any two of these values, relative to each of the other non-CRV431 components (including, but not limited to, Vitamin E, Maisine^{®} CC, propylene glycol, Transcutol^{®}, and Cremophor^{®} RH40) in the SMEDDS.

The SMEDDS of the present disclosure comprises Cremophor^{®} RH40 at a weight ratio of between about 4 and about 8 (e.g., about 4, about 4.25, about 4.5, about 4.75, about 5, about 5.25, about 5.5, about 5.75, about 6, about 6.25, about 6.5, about 6.75, about 7, about 7.25, about 7.5, about 7.75, about 8) relative to the non-CRV431 components in the SMEDDS. In particular, the SEMDDS comprises Cremophor^{®} RH40 at a weight ratio of between about 4 and about 8 relative to each of the Vitamin E, Maisine^{®} CC, propylene glycol, Transcutol^{®}, and ethanol in the SMEDDS. In preferred embodiments, the SMEDDS of the present disclosure may comprise Cremophor^{®} RH40 at a weight ratio of between about 4 and about 6. In some embodiments, the SMEDDS can comprise Cremophor^{®} RH40 at a weight ratio of, of about, of at least, or of at most, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, or a number or a range between any two of these values, relative to each of the other non-CRV431 components (including, but not limited to, Vitamin E, Maisine^{®} CC, propylene glycol, Transcutol^{®}, and Cremophor^{®} RH40) in the SMEDDS. According to the invention, the SMEDDS comprises Vitamin E, Maisine^{®} CC, propylene glycol, Transcutol^{®}, ethanol, and Cremophor^{®} RH40 at a weight ratio of about (0.75-1.5)/(0.5-2)/(2-5)/(2-5)/(2-2.4)/(4-8).

In some embodiments, the SMEDDS of the present disclosure can comprise CRV431 at a concentration of between about 1 mg/mL and about 100 mg/mL (e.g., about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL). In preferred embodiments the SMEDDS of the present disclosure may comprise CRV431 at a concentration of about 10 mg/mL to about 90 mg/mL. In some embodiments, the SMEDDS can comprise CRV431 at a concentration of, of about, of at least, or of at most, 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 31 mg/mL, 32 mg/mL, 33 mg/mL, 34 mg/mL, 35 mg/mL, 36 mg/mL, 37 mg/mL, 38 mg/mL, 39 mg/mL, 40 mg/mL, 41 mg/mL, 42 mg/mL, 43 mg/mL, 44 mg/mL, 45 mg/mL, 46 mg/mL, 47 mg/mL, 48 mg/mL, 49 mg/mL, 50 mg/mL, 51 mg/mL, 52 mg/mL, 53 mg/mL, 54 mg/mL, 55 mg/mL, 56 mg/mL, 57 mg/mL, 58 mg/mL, 59 mg/mL, 60 mg/mL, 61 mg/mL, 62 mg/mL, 63 mg/mL, 64 mg/mL, 65 mg/mL, 66 mg/mL, 67 mg/mL, 68 mg/mL, 69 mg/mL, 70 mg/mL, 71 mg/mL, 72 mg/mL, 73 mg/mL, 74 mg/mL, 75 mg/mL, 76 mg/mL, 77 mg/mL, 78 mg/mL, 79 mg/mL, 80 mg/mL, 81 mg/mL, 82 mg/mL, 83 mg/mL, 84 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, or a number or a range between any two of these values.

In some embodiments, the weight ratio of the components of the SMEDDS other than CRV431 is with respect to other components of the SMEDDS other than CRV431. In some embodiments, the SMEDDS of the disclosure is prepared by combining (e.g., mixing) components of the SMEDDS other than CRV431 (e.g., Vitamin E, Maisine^{®} CC, propylene glycol, Transcutol^{®}, ethanol, and Cremophor^{®} RH40) at a weight ratio disclosed herein into a mixture before combining CRV431 with the mixture (e.g., dissolving CRV431 into the mixture, or adding the mixture to CRV431) to obtain the SMEDDS with CRV431 of any concentration (e.g., 90 mg/mL) disclosed herein. In some embodiments, the SMEDDS of the disclosure is prepared by combining (e.g., mixing) the components of the SMEDDS at once.

In some embodiments, the SMEDDS is stable at room temperature. In some embodiments, the SMEDDS is stable at 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 45°C, 50°C, 55°C, 60°C, or a number or a range between any two of these values.

The SMEDDS disclosed herein is stable in storage. For example, the SMEDDS can be stable for a few hours, a few days, a few months, or a few years. In some embodiments, the SMEDDS is stable for about one month to about three years. In some embodiments, the SMEDDS is stable for from at least about 25 days to at least about a year. The SMEDDS can be stable for, for about, for at least, or for at most, 25 days, 26 days, 27 days, 28 days, 29 days, 30 days, 31 days, 32 days, 33 days, 34 days, 35 days, 36 days, 37 days, 38 days, 39 days, 40 days, 41 days, 42 days, 43 days, 44 days, 45 days, 46 days, 47 days, 48 days, 49 days, 50 days, 51 days, 52 days, 53 days, 54 days, 55 days, 56 days, 57 days, 58 days, 59 days, 60 days, 61 days, 62 days, 63 days, 64 days, 65 days, 66 days, 67 days, 68 days, 69 days, 70 days, 71 days, 72 days, 73 days, 74 days, 75 days, 76 days, 77 days, 78 days, 79 days, 80 days, 81 days, 82 days, 83 days, 84 days, 85 days, 86 days, 87 days, 88 days, 89 days, 90 days, 91 days, 92 days, 93 days, 94 days, 95 days, 96 days, 97 days, 98 days, 99 days, 100 days, 111 days, 112 days, 113 days, 114 days, 115 days, 116 days, 117 days, 118 days, 119 days, 120 days, 121 days, 122 days, 123 days, 124 days, 125 days, 126 days, 127 days, 128 days, 129 days, 130 days, 131 days, 132 days, 133 days, 134 days, 135 days, 136 days, 137 days, 138 days, 139 days, 140 days, 141 days, 142 days, 143 days, 144 days, 145 days, 146 days, 147 days, 148 days, 149 days, 150 days, 51 days, 152 days, 153 days, 154 days, 155 days, 156 days, 157 days, 158 days, 159 days, 160 days, 161 days, 162 days, 163 days, 164 days, 165 days, 166 days, 167 days, 168 days, 169 days, 170 days, 171 days, 172 days, 173 days, 174 days, 175 days, 176 days, 177 days, 178 days, 179 days, 180 days, 181 days, 182 days, 183 days, 184 days, 185 days, 186 days, 187 days, 188 days, 189 days, 190 days, 191 days, 192 days, 193 days, 194 days, 195 days, 196 days, 197 days, 198 days, 199 days, 200 days, or a number or a range between any two of these values. The SMEDDS can be stable for, for about, for at least, or for at most, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 2.5 years, 3 years, or a number or a range between any two of these values. The SMEDDS can be considered stable, for example, if CRV431 does not crystallize, or precipitate, substantially, and/or to an extent that is visually observable.

The SMEDDS can be considered stable, for example, if CRV431 in the SMEDDS does not crystallize, or precipitate, to such an extent that the concentration of CRV431 solubilized in the SMEDDS decreases by more than 0.1%, 0.2%, 0.3%, 4%, 5%, 6%, 7%, 8%, 9%, 1%, 2%, 3%, 4%, 5%, 6%, 7%, 8%, 9%, 10%, or a number or a range between any two of these values. The SMEDDS can be considered stable, for example, if CRV431 in the SMEDDS does not crystallize, or precipitate, to such an extent that the concentration of CRV431 solubilized in the SMEDDS decreases by more than 0.1 mg/ml, 0.2 mg/ml, 0.3 mg/ml, 4 mg/ml, 5 mg/ml, 6 mg/ml, 7 mg/ml, 8 mg/ml, 9 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, or a number or a range between any two of these values.

In some embodiments, the diameter of particles formed by the SMEDDS dispersed in an aqueous solution is from about 15 nm to about 40 nm. In some embodiments, the diameter of particles formed by the SMEDDS dispersed in an aqueous solution is, is about, is at least, or is at most, 15 nm, 16 nm, 17 nm, 18 nm, 19 nm, 20 nm, 21 nm, 22 nm, 23 nm, 24 nm, 25 nm, 26 nm, 27 nm, 28 nm, 29 nm, 30 nm, 31 nm, 32 nm, 33 nm, 34 nm, 35 nm, 36 nm, 37 nm, 38 nm, 39 nm, 40 nm, or a number or a range between any two of these values.

### Use of the Pharmaceutical Compositions of the Disclosure

An active compound of the present disclosure (e.g., as part of a SMEDDS composition) may optionally be administered in combination (or in conjunction) with one or more other active compounds and/or agents useful in the treatment of viral infections as described herein. The administration of two or more compounds "in combination" or "in conjunction" means that the two or more compounds are administered closely enough in time to have a combined effect, for example an additive and/or synergistic effect. The two or more compounds may be administered simultaneously (concurrently) or sequentially or there may be two or more events occurring within a short time period before or after each other. Simultaneous administration may be carried out by mixing the compounds prior to administration, or by administering the compounds at the same point in time but at different anatomic sites or using different routes of administration. In some embodiments, the other antiviral agent(s) may optionally be administered concurrently.

For example, in some embodiments the pharmaceutical compositions (e.g., SMEDDS compositions) of the present disclosure may be administered in temporal proximity with another active agent (e.g., another antiviral agent or a booster agent). In some embodiments, "temporal proximity" means that administration of one therapeutic agent (e.g., a pharmaceutical composition of the present disclosure) occurs within a time period before or after the administration of another therapeutic agent (e.g., an additional antiviral agent), such that the therapeutic effect of the one therapeutic agent overlaps with the therapeutic effect of the other therapeutic agent. In some embodiments, the therapeutic effect of the one therapeutic agent completely overlaps with the therapeutic effect of the other therapeutic agent. In some embodiments, "temporal proximity" means that administration of one therapeutic agent occurs within a time period before or after the administration of another therapeutic agent, such that there is a synergistic effect between the one therapeutic agent and the other therapeutic agent. "Temporal proximity" may vary according to various factors, including but not limited to, the age, gender, weight, genetic background, medical condition, disease history, and treatment history of the subject to which the therapeutic agents are to be administered; the disease or condition to be treated or ameliorated; the therapeutic outcome to be achieved; the dosage, dosing frequency, and dosing duration of the therapeutic agents; the pharmacokinetics and pharmacodynamics of the therapeutic agents; and the route(s) through which the therapeutic agents are administered. In some embodiments, "temporal proximity" means within 15 minutes, within 30 minutes, within an hour, within two hours, within four hours, within six hours, within eight hours, within 12 hours, within 18 hours, within 24 hours, within 36 hours, within 2 days, within 3 days, within 4 days, within 5 days, within 6 days, within a week, within 2 weeks, within 3 weeks, within 4 weeks, with 6 weeks, or within 8 weeks. In some embodiments, multiple administration of one therapeutic agent can occur in temporal proximity to a single administration of another therapeutic agent. In some embodiments, temporal proximity may change during a treatment cycle or within a dosing regimen.

In some embodiments, the SMEDDS of the present disclosure can be used to treat a disease, e.g., a liver disease, such as a severe liver disease, hepatitis B (HBV), liver fibrosis or hepatocellular carcinoma. Non-limiting examples of liver disease include intrahepatic cholestasis (alagille syndrome, biliary liver cirrhosis), fatty liver (alcoholic fatty liver, reye syndrome), hepatic vein thrombosis, hepatolentricular degeneration, hepatomegaly, liver abscess (amebic liver abscess), liver cirrhosis (alcoholic, biliary and experimental), alcoholic liver diseases (fatty liver, hepatitis, cirrhosis), parasitic (hepatic echinococcosis, fascioliasis, amebic liver abscess), jaundice (hemolytic, hepatocellular, and cholestatic), cholestasis, portal hypertension, liver enlargement, ascites, hepatitis (alcoholic hepatitis, animal hepatitis, chronic hepatitis (autoimmune, hepatitis B, hepatitis C, hepatitis D, drug induced), toxic hepatitis, viral human hepatitis (hepatitis A, hepatitis B, hepatitis C, hepatitis D, hepatitis E), Wilson's disease, granulomatous hepatitis, secondary biliary cirrhosis, hepatic encephalopathy, varices, primary biliary cirrhosis, primary sclerosing cholangitis, hepatocellular adenoma, hemangiomas, bile stones, liver failure (hepatic encephalopathy, acute liver failure), and liver neoplasms (angiomyolipoma, calcified liver metastases, cystic liver metastases, epithelial tumors, fibrolamellar hepatocarcinoma, focal nodular hyperplasia, hepatic adenoma, hepatobiliary cystadenoma, hepatoblastoma, hepatocellular carcinoma, hepatoma, liver cancer, liver hemangioendothelioma, mesenchymal hamartoma, mesenchymal tumors of liver, nodular regenerative hyperplasia, benign liver tumors (Hepatic cysts [Simple cysts, Polycystic liver disease, Hepatobiliary cystadenoma, Choledochal cyst], Mesenchymal tumors [Mesenchymal hamartoma, Infantile hemangioendothelioma, Hemangioma, Peliosis hepatis, Lipomas, Inflammatory pseudotumor, Miscellaneous], Epithelial tumors [Bile duct epithelium (Bile duct hamartoma, Bile duct adenoma), Hepatocyte (Adenoma, Focal nodular hyperplasia, Nodular regenerative hyperplasia)], malignant liver tumors [hepatocellular, hepatoblastoma, hepatocellular carcinoma, cholangiocellular, cholangiocarcinoma, cystadenocarcinoma, tumors of blood vessels, angiosarcoma, Karposi's sarcoma, hemangioendothelioma, embryonal sarcoma, fibrosarcoma in liver, leiomyosarcoma, rhabdomyosarcoma, carcinosarcoma, teratoma, carcinoid, squamous carcinoma, primary lymphoma]), peliosis hepatis, erythrohepatic porphyria, hepatic porphyria (acute intermittent porphyria, porphyria cutanea tarda), Zellweger syndrome). In some embodiments, the liver disease is hepatitis, cirrhosis, cholestasis or liver failure.

In some embodiments, during or after the treatment, a symptom of the disease in the subject is alleviated and/or a severity of the disease in the subject decreases. Non-limiting examples of the symptom includes, skin and eyes that appear yellowish (jaundice), abdominal pain and swelling, swelling in the legs and ankles, itchy skin, dark urine color, pale stool color, or bloody or tar-colored stool, chronic fatigue, nausea or vomiting, loss of appetite, tendency to bruise easily. The severity of the disease can increase (or decrease) by 1, 2, 3, 4, 5, 6, 7, 8, 9, 10, 11, 12, 13, 14, 15, 16, 17, 18, 19, 20, 25, 30, 40, 50, 60, 70, 80, 90, 100, or a number of a range between any two of these values, disease severity score unit (e.g., model for end-stage liver disease (MELD score) unit. In some embodiments, during or after the treatment, a function of a liver of the subject is improved. For example, the function of the liver of the subject is improved by 5%, 10%, 15%, 20%, 25%, 30%, 40%, 50%, 60%, 70%, 80% 90%, 100%, or a number or a range between any two of these values. In some embodiments, during or after the treatment, a load of a virus causing the disease decreases. For example, the load of the virus causing the disease can decrease by, by about, by at least, or by at most, 5%, 10%, 15%, 20%, 25%, 30%, 40%, 50%, 60%, 70%, 80% 90%, 95%, 96%, 97%, 98%, 99%, 100%, or a number or a range between any two of these values.

With respect to treatment of diseases or disorders, the "effective amount" is determined with reference to the recommended dosages of the SMEDDS. The selected dosage will vary depending on the activity of the selected compound, the route of administration, the severity of the condition being treated, and the condition and prior medical history of the patient being treated. However, it is within the skill of the art to start doses of the compound(s) at levels lower than required to achieve the desired therapeutic effect and to gradually increase the dosage until the desired effect is achieved. If desired, the effective daily dose may be divided into multiple doses for purposes of administration, for example, two to four doses per day. It will be understood, however, that the specific dose level for any particular patient will depend on a variety of factors, including the body weight, general health, diet, time, and route of administration and combination with other drugs, and the severity of the disease being treated.

In some embodiments, the SMEDDS of the disclosure is administered e.g., at a dosage of from about 1 mg/kg to about 13 mg/kg, for example at a dosage of from about 1 mg/kg to about 10 mg/kg. For example, said compound can be administered to said subject at a dosage of, or about, of at least, or of at most, 1 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, 5 mg/kg, 6 mg/kg, 7 mg/kg, 8 mg/kg, 9 mg/kg, 10 mg/kg, 11 mg/kg, 12 mg/kg, 13 mg/kg, , or a number or a range between any two of these values. Alternatively, or in addition, said compound is administered to said subject in an amount of, of about, of at least, or of at most, 25 mg, 50 mg, 75 mg, 100 mg, 125 mg, 150 mg, 175 mg, 200 mg, 250 mg, 300 mg, 350 mg, 400 mg, 450 mg, 500 mg, 600 mg, 700 mg, 800 mg, 900 mg, 1000 mg, or a number or a range between any two of these values. The SMEDDS of the disclosure can be administered, for example, as a single dose, daily, or weekly. In any of the embodiments disclosed herein, the SMEDDS can be administered orally.

The number of dosages per course of treatment can be different. In some embodiments, the number of dosages per course of treatment can be, be about, be at least, or be at most, 1, 2, 3, 4, 5, 6, 7, 8, 9, 10, 11, 12, 13, 14, 15, 16, 17, 18, 19, 20, or a number or a range between any two of these values. A dosage can be administered a number of days, a number of weeks, or a number of months, after the immediate prior dosage, such as 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 12 months, one year, two years, three years, four years, five years, or more, or a number or a range between any two of these values.

The number of courses of treatment a subject receives can be different. In some embodiments, the number of courses of treatment can be, be about, be at least, or be at most, 1, 2, 3, 4, 5, 6, 7, 8, 9, 10, 11, 12, 13, 14, 15, 16, 17, 18, 19, 20, or a number or a range between any two of these values. A course of treatment can be administered a number of days, a number of weeks, or a number of months, after the immediate prior course of treatment, such as 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 12 months, or a number or a range between any two of these values.

In some embodiments, the SMEDDS describe herein can be administered, for example, as a single dose, weekly, or every other week, or every three weeks, or monthly. The SMEDDS of the disclosure can be administered once per day for 1, 2, 3, 4, 5, 6, 7, 8, 9, or 10 days or more. For example, a SMEDDS comprising 5 mg of CRV431 can be administered daily. For example, a SMEDDS comprising 10 mg of CRV431 can be administered daily. For example, a SMEDDS comprising 15 mg of CRV431 can be administered daily. For example, a SMEDDS comprising 20 mg of CRV431 can be administered daily. For example, a SMEDDS comprising 25 mg of CRV431 can be administered daily. For example, a SMEDDS comprising 50 mg of CRV431 can be administered daily. For example, a SMEDDS comprising 100 mg of CRV431 can be administered daily. For example, a SMEDDS comprising 150 mg of CRV431 can be administered daily. For example, a SMEDDS comprising 200 mg of CRV431 can be administered daily. For example, a SMEDDS comprising 300 mg of CRV431 can be administered daily. For example, a SMEDDS comprising 350 mg of CRV431 can be administered daily. For example, a SMEDDS comprising 400 mg of CRV431 can be administered daily. For example, a SMEDDS comprising 450 mg of CRV431 can be administered daily. For example, a SMEDDS comprising 500 mg of CRV431 can be administered daily. For example, a SMEDDS comprising 750 mg of CRV431 can be administered daily. For example, a SMEDDS comprising 1000 mg of CRV431 can be administered daily. The SMEDDS of the disclosure can be administered once per day for 1, 2, 3, 4, 5, 6, 7 days, or more. The SMEDDS of the disclosure can be administered once per day for 1, 2, 3, 4, 5, 6, 7, 8, 9, or 10 weeks or more. For example, a SMEDDS comprising 5 mg of CRV431 can be administered weekly. For example, a SMEDDS comprising 10 mg of CRV431 can be administered weekly. For example, a SMEDDS comprising 15 mg of CRV431 can be administered weekly. For example, a SMEDDS comprising 20 mg of CRV431 can be administered weekly. For example, a SMEDDS comprising 25 mg of CRV431 can be administered weekly. For example, a SMEDDS comprising 50 mg of CRV431 can be administered weekly. For example, a SMEDDS comprising 100 mg of CRV431 can be administered weekly. For example, a SMEDDS comprising 150 mg of CRV431 can be administered weekly. For example, a SMEDDS comprising 200 mg of CRV431 can be administered weekly. For example, a SMEDDS comprising 300 mg of CRV431 can be administered weekly. For example, a SMEDDS comprising 350 mg of CRV431 can be administered weekly. For example, a SMEDDS comprising 400 mg of CRV431 can be administered weekly. For example, a SMEDDS comprising 450 mg of CRV431 can be administered weekly. For example, a SMEDDS comprising 500 mg of CRV431 can be administered weekly. For example, a SMEDDS comprising 750 mg of CRV431 can be administered weekly. For example, a SMEDDS comprising 1000 mg of CRV431 can be administered weekly. For example, a SMEDDS comprising 1250 mg of CRV431 can be administered weekly. For example, a SMEDDS comprising 1500 mg of CRV431 can be administered weekly. For example, a SMEDDS comprising 1750 mg of CRV431 can be administered weekly. For example, a SMEDDS comprising 2000 mg of CRV431 can be administered weekly.

In some embodiments, after a dosage is administered, an area under curve (AUC) of a plot of a concentration of CRV431 in a blood of the subject over time is from about 5000 ng.hr/ml to about 150000 ng.hr/ml. The AUC of the plot of the concentration of CRV431 in the blood of the subject over time can be, be about, be at least, or be at most, 5000 ng.hr/ml, 10000 ng.hr/ml, 11000 ng.hr/ml, 12000 ng.hr/ml, 13000 ng.hr/ml, 14000 ng.hr/ml, 15000 ng.hr/ml, 16000 ng.hr/ml, 17000 ng.hr/ml, 18000 ng.hr/ml, 19000 ng.hr/ml, 20000 ng.hr/ml, 30000 ng.hr/ml, 40000 ng.hr/ml, 50000 ng.hr/ml, 60000 ng.hr/ml, 70000 ng.hr/ml, 80000 ng.hr/ml, 90000 ng.hr/ml, 100000 ng.hr/ml, 110000 ng.hr/ml, 120000 ng.hr/ml, 130000 ng.hr/ml, 140000 ng.hr/ml, 150000 ng.hr/ml, or a number or a range between any two of these values.

In some embodiments, a maximum concentration (Cₘₐₓ) of CRV431 in the blood of the subject after a single dosage (for example, a single dosage disclosed herein, including a single dose of CRV431 at 1 mg/kg or at 3 mg/kg, or a number or a range between 1 mg/kg or 3 mg/kg) is administered is from about 1500 ng/ml to about 2500 ng/ml. For example, the Cₘₐₓ can be, be about, be at least, or be at most, 1500 ng/ml, 1550 ng/ml, 1600 ng/ml, 1650 ng/ml, 1700 ng/ml, 1750 ng/ml, 1800 ng/ml, 1850 ng/ml, 1900 ng/ml, 1950 ng/ml, 2000 ng/ml, 2050 ng/ml, 2100 ng/ml, 2150 ng/ml, 2200 ng/ml, 2250 ng/ml, 2300 ng/ml, 2350 ng/ml, 2400 ng/ml, 2450 ng/ml, 2500 ng/ml, or a number or a range between any two of these values. In some embodiments, Cₘₐₓ of CRV431 in the blood of the subject is measured after multiple dosages of the SMEDDS of the disclosure. For example, the Cₘₐₓ after multiple dosing can be, be about, be at least, or be at most, 1500 ng/ml, 1550 ng/ml, 1600 ng/ml, 1650 ng/ml, 1700 ng/ml, 1750 ng/ml, 1800 ng/ml, 1850 ng/ml, 1900 ng/ml, 1950 ng/ml, 2000 ng/ml, 2050 ng/ml, 2100 ng/ml, 2150 ng/ml, 2200 ng/ml, 2250 ng/ml, 2300 ng/ml, 2350 ng/ml, 2400 ng/ml, 2450 ng/ml, 2500 ng/ml, 3000 ng/ml, 3500 ng/ml, 4000 ng/ml, 4500 ng/ml, 5000 ng/ml, or a number or a range between any two of these values.

In some embodiments, a time (Tₘₐₓ) to reach a maximum concentration of CRV431 after a dosage is administered in a blood of the subject is from about 0.5 hour to about 8 hours. The Tₘₐₓ can be, be about, be at least, or be at most, 0.5 hr, 1 hr, 1.5 hrs, 2 hrs, 2.5 hrs, 3 hrs, 3.5 hrs, 4 hrs, 4.5 hrs, 5 hrs, 5.5 hrs, 6 hrs, 6.5 hrs, 7 hrs, 7.5 hrs, 8 hrs, or a number or a range between any two of these values.

In some embodiments, an elimination half-life (T_{1/2}) of CRV431 in a blood of the subject is from about 10 hours to about 200 hours. The T_{1/2} can be, be about, be at least, or be at most, 10 hrs, 11 hrs, 12 hrs, 13 hrs, 14 hrs, 15 hrs, 16 hrs, 17 hrs, 18 hrs, 19 hrs, 20 hrs, 25 hrs, 30 hrs, 35 hrs, 40 hrs, 45 hrs, 50 hrs, 60 hrs, 70 hrs, 80 hrs, 90 hrs, 100 hrs, 110 hrs, 120 hrs, 130 hrs, 140 hrs, 150 hrs, 160 hrs, 170 hrs, 180 hrs, 190 hrs, 200 hrs, or a number or a range between any two of these values.

In some embodiments, a concentration of CRV431 in a liver of the subject relative to a concentration of CRV431 in a blood of the subject is from about 1 to about 20.

### EXAMPLES

The disclosure is further illustrated by the following examples. It is to be understood that the examples are provided to illustrate certain embodiments and that no limitation to the scope of the disclosure is intended thereby.

### Example 1 - Development of a Self-Microemulsifying Drug Delivery System

### Drugs and reagents

CRV431 was synthesized to a purity of 97.3 % by modification of CsA and stored at 5°C. CsA was obtained from *IVAX* (Opava, Czech Republic). Lauroglycol^{™} 90, Labrasol^{®}, Labrafil^{®} M 2125, Labrafil^{®} 1944, Peceol^{™}, Labrafac^{™} WL 1349, Capryol^{™} 90, Maisine^{®} CC, and Transcutol^{®} were purchased from *Gattefosse* (Montreal, Canada). Propylene glycol, filtered water, acetonitrile, methanol, scintillation vials and 12x75 ml borosilicate test tubes were purchased from *Fischer Scientific* (Pittsburgh, USA). Anhydrous ethanol was purchased from *Commercial Alcohols* (Toronto, Canada). Vitamin E, Tween^{®} 80, Tween^{®} 40, Tween^{®} 20, castor oil, dimethyl sulfoxide, Chremophor^{®} EL and Chremophor RH 40 were purchased from *Sigma-Aldrich* (St. Louis, USA). Span^{®} 80 was purchased from *EMD Millipore Corporation* (Burlington, USA). Polyethylene glycol 400 (PEG 400) was purchased from *BDH Incorporated* (Mississauga, Canada).

### Physicochemical properties

To determine the aqueous solubilities of CRV431 and CsA, drug stocks at 20 mM in dimethyl sulfoxide (DMSO) were diluted 30-fold to achieve a final concentration of 666 µM in phosphate buffered saline (PBS, pH 7.4), placed on a shaker overnight, and centrifuged at 3,300 rpm for 5 min to pellet insoluble drug. The supernatant was collected, diluted 1:1 with methanol, and drug concentrations measured by HPLC (Agilent 1100 series, UV detection) relative to standard curves. The polar surface area and log P were calculated using Molinspiration Cheminformatics (V2018.10, USA). Plasma protein binding was determined by equilibrium dialysis assays. Drug plasma samples were prepared to achieve a final concentration of 5 µM in human blood plasma by 500-fold dilution of 2.5 mM DMSO-drug stocks. Plasma solutions (300 µl) and PBS, pH 7.4 (500 µl), were added to adjacent chambers of Rapid Equilibrium Dialysis units (*Thermo Scientific*; 8K MWCO). Plates were sealed with plastic film and shaken on an orbital shaker at 225 rpm at 37°C for 4.5 hr. Plasma and PBS samples were collected, and drugs extracted with a zinc sulfate/methanol precipitation method. After centrifugation at 3,300 rpm for 10 min, soluble drugs in methanol supernatants were analyzed by Liquid Chromatography (LC) Electrospray Ionization (ESI) Mass Spectrometry (LC-ESI-MS) (Agilent 1100 series, Santa Clara, USA) and quantified relative to standard curves.

### Excipient Solubility

The solubility of CRV431 was measured in groups of surfactants, oils, and co-solvents for the preparation of SMEDDS formulations suitable for use in water dispersions and softgel capsules. CRV431 (50 mg) was added to the bottom of a 75x125 mm glass test tube and brought up to the 1 ml mark with excipient. The sample was placed on a rocker and visually assessed for solubility after overnight mixing. If solubility was not achieved, excipient was added in 0.5 ml increments, with subsequent overnight mixing, until a clear solution was reached. Visual assessment was used for solubility differentiation among the excipients, and relative to CsA.

### Construction of Pseudo-Ternary Phase Diagrams

For construction of a pseudo-ternary phase diagram, mixtures containing different compositions of Cremophor^{®} RH40 (surfactant), Maisine^{®} CC (oil) and co-solvents (Vitamin E, propylene glycol, Transcutol^{®} and ethanol) were evaluated to assess the phase boundary between microemulsion formation (clear to the eye) and non-microemulsion formations (cloudy to the eye) for the various excipient ratios.

### CRV431 SMEDDS Development

CRV431 (50-100 mg) was added to the bottom of a 75x125 mm glass test tube. Excipients (e.g., Vitamin E, Maisine^{®} CC, propylene glycol, Transcutol^{®}, ethanol, and Cremophor^{®} RH40) were separately combined and mixed well prior to adding to CRV431 and brought up to the 1 ml mark. Several series of SMEDDS formulations were prepared and assessed for drug solubility, stability (by LC-ESI-MS), and microemulsion formation and stability (visual inspection) in aqueous media.

### LC-ESI-MS Quantitation of CRV431 in SMEDDS Formulations

For all SMEDDS formulation analysis 10 µl was removed and added to 10 ml of methanol in a scintillation vial. The sample was vortexed for 10 seconds to mix and 1 ml of this solution was added to 9 ml of methanol in another scintillation vial (total 10,000-fold dilution). A 5 µg/ml CRV431 standard in methanol was also prepared. Samples (1 ml) were transferred to injection vials and analyzed by liquid chromatography-electrospray ionization mass spectrometry (LC-ESI-MS) on an Agilent HP 1100 LC-MS. Samples were placed in an autosampler maintained at 5°C. Samples and the CRV431 standard were injected (1 µl) onto a Zorbax SB-C18 reverse phase HPLC column (1.8 µm Rapid Resolution HT Cartridge, 4.6 x 30 mm) maintained at 75°C using an acetonitrile-water gradient system containing 0.02% glacial acetic acid and 20 µM sodium acetate (Table 1).

**Table 1. Gradient Conditions for Elution of CRV431.**

| Time (min) | dH₂0^{∗} (%) | Acetonitrile (ACN)* | Flow Rate (mL/min) |
|---|---|---|---|
| 0.0 | 55 | 45 | 1.0 |
| 6.0 | 25 | 75 | 1.0 |
| 8.1 | 0 | 100 | 1.0 |
| 10.0 | 0 | 100 | 1.0 |
| 10.1 | 55 | 45 | 1.0 |
| *also contains 0.02% Glacial acetic acid + 20 µM Sodium Acetate | | | |

The sodium-adduct of CRV431 (1326 m/z) was analyzed by mass spectrometry (MS) using electrospray ionization (ESI) in positive ion mode. The ESI-MS was conducted with N₂ gas temperature set at 350°C and drying gas at 12 L/min. The fragmentor and capillary voltages were set at 260 and 4000 volts, respectively. The nebulizer pressure was set at 40 psig. CRV431 elution time was typically observed at 6.0 minutes. The concentration of the formulations was calculated by peak area comparison with the one-point standard (5 µg/ml).

### Dispersion Study

Approximately 5 mL of the selected media (water, 0.1 N HCL, or phosphate buffer (pH 6.8) was placed in a vial and four (4) drops (around 40 mg) of the 1/1/5/5/2.4/4 (Vitamin E/Maisine^{®} CC/Propylene Glycol/Transcutol^{®}/Ethanol/Cremophor^{®} RH40 (w/w/w/w/w/w)) SMEDDS was added. The mixture was observed initially, after inverting, and after one hour of adding the 1/1/5/5/2.4/4 (Vitamin E/Maisine^{®} CC/Propylene Glycol/Transcutol^{®}/Ethanol/ Cremophor^{®} RH40 (w/w/w/w/w/w)) SMEDDS. This same procedure was also conducted on the 1/1.5/2.5/5/2.4/5 SMEDDS.

### Particle size

The mean particle diameter of both the 1/1/5/5/2.4/4 and 1/1.5/2.5/5/2.4/5 (Vitamin E/Maisine^{®} CC/Propylene Glycol/Transcutol^{®}/Ethanol/Cremophor^{®} RH40 (w/w/w/w/w/w)) SMEDDS was measured at 25°C by dynamic light scattering (Zetasizer Nano, Malvern Instruments, Malvern, UK) at an angle of 173°. Each sample was measured in triplicate. Values were expressed as a mean ± standard deviation. Mean value was approximately 25 nm.

### Pharmacokinetics of 1/1.5/2.5/5/2.4/5 (Vitamin E/Maisine^{®} CC/Propylene Glycol/Transcutol^{®}/Ethanol/Cremophor^{®} RH40 (w/w/w/w/w/w)) CRV431 SMEDDS in humans

CRV431 SMEDDS was orally administered to 6 healthy fasted human volunteers as a single dose of either 1 or 3 mg/kg (75 mg or 225 mg). The study was conducted at a clinical research facility for investigational medicines (Celerion, Arizona, USA) as part of a clinical Phase 1 single-ascending-dose (SAD) study and followed all FDA ethical guidelines. Patients were selected according to standardized inclusion/exclusion criteria for a Phase I SAD study. Healthy volunteers 18-55 years of age with no evidence of ongoing disease (as determined by the study investigator), or any use of nicotine (30 days prior to screening), or drugs of abuse (within the preceding 2 years), or use of chronic prescription medication (within 30 days), or acute prescription medication (within 14 days), or systemic over-the-counter medications including vitamins and herbal/natural supplements (within 7 days prior to the study dose) were enrolled in the study. The SMEDDS was dispensed (approximately 3 ml) into a 100 ml Gibco clear bottle and a 15-fold excess of filtered water (HPLC grade) was added. The mixture was swirled gently for 1-2 minutes until fully dispersed. The SMEDDS in-water dose was placed in the refrigerator and orally administered within 2 hours. Whole blood samples (0.5 ml) were drawn by venipuncture into K2-EDTA blood collection tubes at 0.5, 1, 2, 4, 6, 8, 12, 24, 48, and 72 hours. Samples were immediately frozen and analyzed by LC-ESI-MS within 5 days. Briefly, whole blood samples were thawed and extracted using a zinc sulfate/methanol precipitation method. Quantitative analysis was performed by a validated LC-ESI-MS method against a 7-point whole blood standard curve. Non-compartmental analysis was performed to calculate pharmacokinetic parameters (Cₘₐₓ, AUC, t_{1/2}, and Tₘₐₓ) using commercial software (WinNonlin Professional Edition, Version 7.0, Pharsight software).

### Pharmacokinetics of CRV431 SMEDDS in rats

SERIES 1 1/3/3/2/9 (Vitamin E/Maisine^{®} CC/Propylene Glycol/Ethanol/ Cremophor^{®} RH40 (w/w/w/w/w)) CRV431 SMEDDS (50 mg/ml) was administered to Sprague Dawley rats (n = 6) by oral gavage at 30 mg/kg/day for 7 days. At 12 hours post-dose on day 7, the animals were sacrificed and whole blood and livers were extracted and frozen. CRV431 was quantitated in whole blood using LC-ESI-MS (as for the human pharmacokinetic studies). For quantitation of CRV431 in liver, one gram samples of liver tissue were added to 9 ml of homogenizing solution (1% formic acid in water) and homogenized using a TissueLyser (Qiagen) at 30 Hz for 10 minutes. Aliquots were removed and analyzed by LC-ESI-MS as described above.

### Physicochemical properties of CRV431

The physicochemical properties of CRV431 and CsA illustrated in Table 2 indicate that CRV431 has a higher aqueous solubility relative to CsA, which is reflected in a higher plasma free-fraction and higher polar surface area (PSA). The Log P values of both drugs are high. Without wishing to be bound by theory, neither CsA nor CRV431 is suitable for oral development as a dry tablet.

**Table 2. Physiochemical Properties of CRV431 and CsA,**

| COMPOUND | SOLUBILITY IN PBS | MOLINS LOG P | MOLINS PSA | PLASMA FREE FRACTION |
|---|---|---|---|---|
| CRV431 | 115.0 µM | 3.98 | 308 | 7.6% |
| CSA | 31.9 µM | 3.61 | 278 | 0.14 % |

### Excipient solubility

The solubility of CRV431 was measured in groups of surfactants, oils, and co-solvents utilized for the commercial preparation of SMEDDS formulations suitable for use in water dispersions and softgel capsules for oral dosage forms.

### Choosing excipients for SMEDDS development

Based on to the CRV431 solubility in Cremophor^{®} RH40, Cremophor^{®} RH40 was chosen as the surfactant. For the oil phase, CRV431 was found to have the highest solubility in Vitamin E followed by Maisine^{®} CC. While for co-solvents the solubility of CRV431 was highest in ethanol, followed by Transcutol^{®}, and propylene glycol. Accordingly, all of the above excipients were chosen for preliminary formulation prototype development.

**Table 3. Excipient Solubility of CRV431 and CsA at 21 °C**

| Category | Excipient | CRV431 Solubility (mg/ml) | Cyclosporine A Solubility (mg/ml) |
|---|---|---|---|
| Surfactant | Tween^{®} 80 | < 10 | > 100 |
| Surfactant | Tween^{®} 40 | < 10 | > 100 |
| Surfactant | Tween^{®} 20 | < 10 | > 100 |
| Surfactant | Lauroglycol^{™} 90 | 30 | ND |
| Surfactant | Labrasol^{®} | < 10 | ND |
| Surfactant | Cremophor^{®} RH40 | 30 | > 50 |
| Surfactant | Cremophor^{®} EL | < 10 | ND |
| Surfactant | Labrafil^{®} M2125 | < 10 | > 100 |
| Surfactant | Labrafil^{®} 1944 | < 10 | ND |
| Surfactant | Span^{®} 80 | < 10 | ND |
| Surfactant | Capryol^{™} | 25 | ND |
| Oil | Peceol^{™} | < 10 | ND |
| Oil | Miglyol 812 | < 10 | > 100 |
| Oil | Maisine^{®} CC | < 20 | 100 |
| Oil | Vitamin E | 25 | ND |
| Oil | Castor Oil | < 10 | > 100 |
| Oil | Labrafac^{™} WL 1349 | < 10 | > 100 |
| Co-solvent | Propylene glycol | 30 | > 100 |
| Co-solvent | Transcutol^{®} | 40 | ND |
| Co-solvent | PEG 400 | < 10 | > 100 |
| Co-solvent | Ethanol | > 200 | > 200 |
| Co-solvent | Dimethyl sulfoxide | < 50 | > 100 |

The solubility of CRV431 in the surfactants tested (as shown in Table 3) ranged between 10-30 mg/ml, in contrast to CsA, which was at least 50 mg/ml, and in most cases, > 100 mg/ml. Given the high lipophilicity of CRV431, the relative low solubility was a surprising finding. The CRV431 solubility was highest in Cremophor^{®} RH40, Lauroglycol^{™} 90 and Capryol^{™} 90. The solubility of CRV431 in the oils tested was less (generally 10 mg/ml) than CsA (> 100 mg/ml), with the exception of Vitamin E (25 mg/ml). The solubility of CRV431 in various co-solvents was found to be higher (10-50 mg/ml) than the surfactants and oils tested, although the solubility of CsA was also generally greater.

### Development of CRV431 SMEDDS

Preliminary formulation studies involved assessing the impact of various excipient ratios on SMEDDS miscibility and microemulsion formation, in the absence of drug. The study results (Table 4) indicate that Cremophor^{®} RH40/Maisine combination ratios less than 3 do not form microemulsions when dispersed in water. Microemulsion formation was assessed by visual inspection and must be clear to the eye to be considered for continued development. Water dispersions with Cremophor^{®} RH40/Maisine ratios less than 3 were all cloudy. The data is diagrammatically expressed in a pseudo-ternary phase diagram (FIG. 2) wherein the phase boundary for microemulsion formation is clearly demarcated.

**Table 4, Preliminary SMEDDS Studies: Excipient Compatibility and Microemulsion Formation as a function of excipient ratios**

| Cremophor^{®} RH40 | Maisine^{®} CC | Vitamin E | Propylene glycol | Transcutol^{®} | Ethanol | SMEDDS (appearance) | Microemulsion formation |
|---|---|---|---|---|---|---|---|
| 3 | 3 | 0 | 10 | 0 | 2 | Biphasic | NO |
| 3 | 5 | 0 | 8 | 0 | 2 | Biphasic | NO |
| 3 | 9 | 0 | 4 | 0 | 2 | Miscible | NO |
| 6.2 | 5 | 3 | 1.2 | 0 | 2 | Miscible | NO |
| 6.4 | 7.8 | 0 | 1.6 | 0 | 1.6 | Miscible | NO |
| 7.2 | 6 | 1 | 1.2 | 0 | 2 | Miscible | NO |
| 7 | 3 | 0 | 6 | 0 | 2 | Miscible | NO |
| 7 | 4.5 | 0 | 4.5 | 0 | 2 | Miscible | NO |
| 7 | 1 | 0 | 8 | 0 | 2 | Miscible | YES |
| 9 | 3 | 1 | 3 | 0 | 2 | Miscible | YES |
| 10 | 4.2 | 0 | 1.5 | 0 | 2 | Miscible | NO |
| 11 | 4 | 0.5 | 0 | 0 | 2 | Miscible | YES |
| 13 | 1 | 0 | 2 | 0 | 2 | Miscible | YES |

### Development and Stability of CRV431 SMEDDS Formulations: SERIES 1 and 2

### SERIES 1

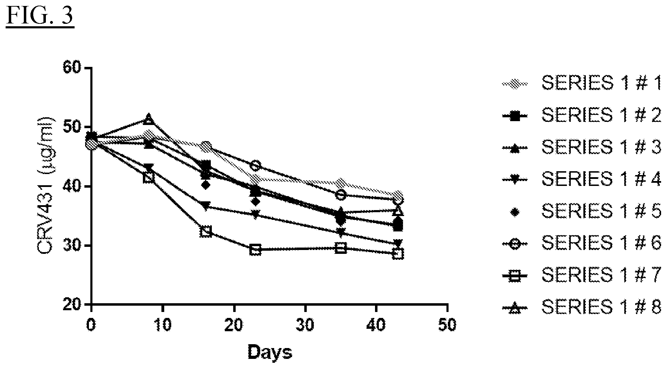

Based on the preliminary SMEDDS microemulsion studies, a prototype SMEDDS formulation (Table 5: formulation # 1) was prepared and assessed for CRV431 solubility and stability. The objective was to produce a miscible and stable SMEDDS formulation which solubilized CRV431 to at least 50 mg/ml, and formed a clear microemulsion in aqueous media. While formulation #1 initially solubilized CRV431 to 50 mg/ml and produced a clear microemulsion, upon subsequent storage, formation of CRV431 crystals adhering to the glass container became apparent within days and coincided with loss of CRV431 as quantitated by LCMS (FIG. 3). Formulation # 1 falls within the upper left circle of the phase diagram in FIG. 2.

**Table 5. Series 1 Excipients and Ratios**

| Formulation # | Vitamin E | Maisine^{®} CC | Propylene glycol | Transcutol^{®} | Ethanol | Cremophor^{®} RH40 |
|---|---|---|---|---|---|---|
| Excipient Ratio by weight | | | | | | |
| 1 | 1 | 3 | 3 | 0 | 2 | 9 |
| 2 | 1 | 3 | 2 | 1 | 2 | 9 |
| 3 | 0.5 | 3 | 2 | 1.5 | 2 | 9 |
| 4 | 0.5 | 3 | 1.5 | 2 | 2 | 9 |
| 5 | 0.5 | 2 | 1.5 | 3 | 2 | 9 |
| 6 | 1 | 2 | 2 | 2 | 2 | 9 |
| 7 | 0.5 | 2 | 0.5 | 4 | 2 | 9 |
| 8 | 1 | 2 | 2.5 | 2.5 | 2 | 8 |

Further SMEDDS were developed (SERIES 1, Table 5) wherein the proportion of Transcutol^{®} co-solvent was increased in an attempt to encourage further solubility and decreased crystal formation. All SERIES 1 formulations were able to develop microemulsions when dispersed in water (displayed within the middle circle of the FIG. 2 phase diagram). The stability results (FIG. 3) however, show that all SERIES 1 SMEDDS are unstable, and that significant CRV431 crystals form upon storage at room temperature.

### SERIES 2 SMEDDS

All SERIES 2 SMEDDS formed clear SMEDDS solutions (Table 6). These formulations are shown to fall within the microemulsion phase of the pseudo-ternary phase diagram (lower right circle of FIG. 2).

**Table 6. SERIES 2 SMEDDS**

| Formulation # | Vitamin E | Maisine^{®} CC | Propylene glycol | Transcutol^{®} | Ethanol | Cremophor^{®} RH40 |
|---|---|---|---|---|---|---|
| 1 | 1.5 | 2 | 2.25 | 2 | 2.25 | 8 |
| 2 | 1.5 | 2 | 2 | 2.25 | 2.25 | 8 |
| 3 | 1.5 | 2 | 2.75 | 2.5 | 2.25 | 7 |
| 4 | 1.5 | 2 | 2.5 | 2.75 | 2.25 | 7 |
| 5 | 1 | 2 | 3.5 | 3.5 | 2 | 6 |
| 6 | 1 | 1 | 5 | 5 | 2 | 4 |
| 6 (75 mg/ml) | 1 | 1 | 5 | 5 | 2 | 4 |
| 7 (70 mg/ml) | 1 | 1.5 | 2.5 | 5 | 2.4 | 5 |

### CRV431 SMEDDS

To further enhance CRV431 solubility, the ethanol content was increased. Tables 7 and 8 demonstrate that when the ethanol weight ratio is increased to 2.4, the drug load of CRV431 can be increased to at least 90 mg/ml and is considered stable. A 100 mg/ml sample was prepared but did not fully solubilize CRV431 and was thus omitted from further testing. All sample preparations were stable when measured after 54 days of storage at room temperature. The CRV431 SMEDDS formulation containing Vitamin E/Maisine^{®} CC/Propylene Glycol/Transcutol^{®}/Ethanol/Cremophor^{®} RH40 in the weight ratios of 1/1/5/5/2.4/4 and 1/1.5/2.5/5/2.4/5 are thus considered CRV431 SMEDDS with high solubility and stability.

**Table 7. Solubility and stability of CRV431 in 1/1/5/5/2.4/4 (Vitamin E/Maisine^{®} CC/Propylene Glycol/Transcutol^{®}/Ethanol/ Cremophor^{®} RH40 (w/w/w/w/w/w)) SMEDDS**

| SMEDDS Formulation: 1/1/5/5/2.4/4 (Vitamin E/Maisine^{®} CC/Propylene Glycol/Transcutol^{®}/Ethanol/ Cremophor^{®} RH40 (w/w/w/w/w/w) | |
|---|---|
| Prepared CRV431 Concentration (mg/ml) | Measured CRV431 Concentration on Day 54 (mg/ml) |
| 75 | 75.2 |
| 80 | 78.6 |
| 90 | 90.0 |

**Table 8. Stability of CRV431 (70 mg/ml) in 1/1.5/2.5/5/2.4/5 (Vitamin E/Maisine^{®} CC/Propylene Glycol/Transcutol^{®}/Ethanol/ Cremophor^{®} RH40 (w/w/w/w/w/w)) SMEDDS**

| Test Description | Specifications | Results (after 3.5 months room temperature storage) |
|---|---|---|
| Assay (mg/g) | Report Results | 70.135 mg/g |
| % Assay | Report Results | 100.2 % |

As shown in FIG. 2, SMEDDS within the top left circled area represent the prototype formulations with high surfactant. SMEDDS within the middle circled area represent SERIES 1 formulations containing high co-solvent amounts. SMEDDS within the bottom right area represent SERIES 2 formulations with low surfactant and high co-solvent amounts. As shown in Table 6, all formulations contained CRV431 at 50 mg/ml, except where indicated. Aliquots of SMEDDS formulations were removed at the indicated times and analyzed by LCMS. All SMEDDS from SERIES 2 were shown to have extended stability (FIG. 4) relative to the original SERIES 1 SMEDDS prototype (1/3/3/9/2). SERIES 2 SMEDDS have no observable loss of drug after 77 days storage at room temperature.

### Characterization of CRV431 SMEDDS formulations (1/1/5/5/2.4/4 and 1/1.5/2.5/5/2.4/5 (Vitamin E/Maisine^{®} CC/Propylene Glycol/Transcutol^{®}/Ethanol/ Cremophor^{®} RH40 (w/w/w/w/w/w))

### Microemulsion Formation and Stability of CRV431 SMEDDS

When CRV431 SMEDDS (1/1/5/5/2.4/4 and 1/1.5/2.5/5/2.4/5 (Vitamin E/Maisine^{®} CC/Propylene Glycol/Transcutol^{®}/Ethanol/Cremophor^{®} RH40 (w/w/w/w/w/w)) were dispersed in aqueous media, a visually clear solution is formed within less than one minute of gentle swirling and/or inversion. Water, 0.1 N HCL, and phosphate buffer microemulsion solutions remained clear for up to 1 hour at 37°C (Table 9).

**Table 9. Microemulsion Stability in Aqueous Media at 37 ° C for 1/1.5/2.5/5/2.2/5 and 1/1/5/5/2.4/4 CRV431 SMEDDS**

| Timepoint | Observations (Visual) | | |
|---|---|---|---|
| | Water | 0.1 N HCL | Phosphate Buffer (pH = 6.8) |
| Initial | Tiny droplets dispersed throughout | Tiny droplets dispersed throughout | Tiny droplets dispersed throughout |
| After swirling and inversion | Clear transparent solution | Clear transparent solution | Clear transparent solution |
| After 1 Hour | Clear transparent solution | Clear transparent solution | Clear transparent solution |

### Particle size

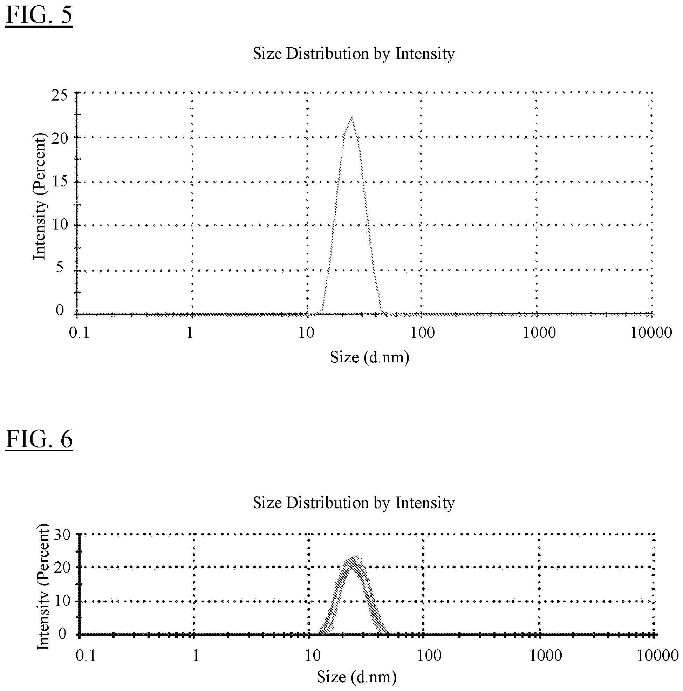

The mean particle diameter of the 1/1.5/2.5/5/2.4/5 CRV431 SMEDDS formulation when dispersed in water was measured to be 24.6 ± 5.7 nm (mean ± standard deviation) (FIG. 5). This size is consistent with the reported particle size of Neoral^{®} aqueous dispersions. Additionally, the 1/1/5/5/2.4/4 SMEDDS formulation has approximately the same particle size (FIG. 6).

### Pharmacokinetics of CRV431 SMEDDS

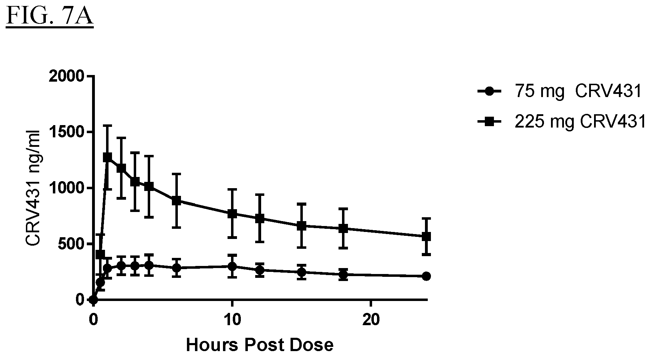

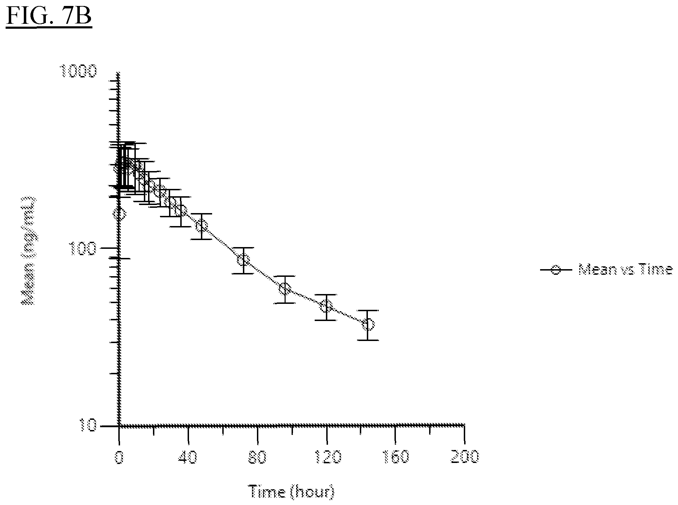

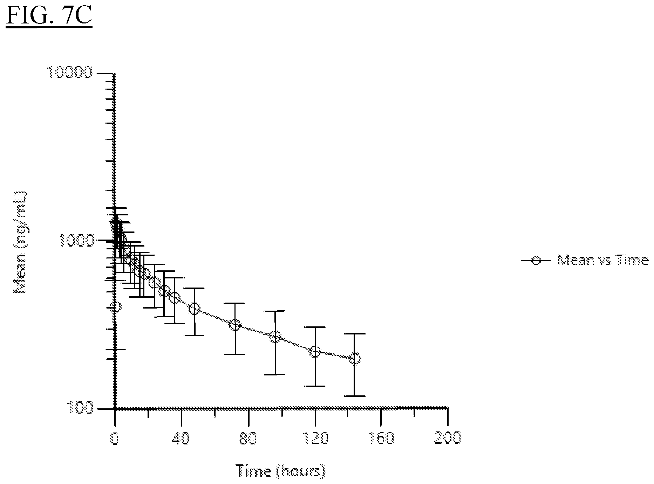

The pharmacokinetics of the 1/1.5/2.5/5/2.4/5 CRV431 SMEDDS in humans demonstrated excellent exposure and approximate dose proportionality (FIGS. 7A-7C and Table 10). Patients (n = 6) were given a single dose of CRV431 and whole blood aliquots analyzed by LC-ESI-MS at the indicated time points. Error bars represent mean ± standard deviation. (FIG. 7A: Linear scale. FIG. 7B: Log scale for the 75 mg dose. FIG. 7C: log scale for the 225 mg dose.) Relative to reported cyclosporine A (2.5 mg/kg) exposures in healthy human subjects (23), CRV431 Cₘₐₓ was slightly greater than Neoral^{®}, and overall exposure (AUC) was approximately 14 times greater than Neoral^{®}.

**Table 10. Single-dose pharmacokinetics of CRV431 in 6 healthy human volunteers relative to cyclosporine A. Values represent mean ± standard deviation**

| Dosing Group | Dose (mg/kg) | AUC _{0-∞} (ng.hr/ml) | Cₘₐₓ (ng/ml) | Tₘₐₓ (Hours) | t_{1/2} (Hours) |
|---|---|---|---|---|---|
| CRV431 | 1 | 20,916 ± 3,780 | 334 ± 106 | 4 ± 3.09 | 73.6 ± 15.2 |
| CRV431 | 3 | 84,421 ± 32,373 | 1,368 ± 221 | 1.33 ± 0.52 | 97.4 ± 18.4 |
| Cyclosporine A | 2.5 | 4,981 ± 1,584 | 944 ± 244 | 1.67 ± 0.48 | 6.3 - 20.4 |

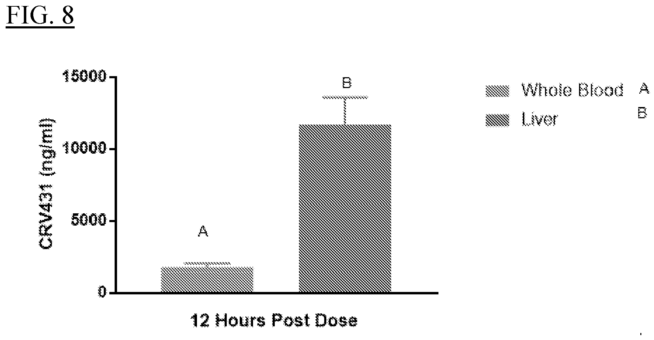

A separate rat study measured the level of CRV431 distributed in the liver vs. the blood (See FIG. 8). In this study, Sprague Dawley rats (n = 6) were dosed (30 mg/kg) with 1/3/3/2/9 (Vitamin E/Maisine^{®} CC/Propylene Glycol/Ethanol/Cremophor^{®} RH40 (w/w/w/w/w)) CRV431 SMEDDS (50 mg/ml) by oral gavage for 7 days, and whole blood aliquots and liver homogenates were analyzed by LC-ESI-MS 12 hours after the final dose. The level of CRV431 distributed in the liver (10 µg/g tissue) was found to be 6.5-fold greater than that in the whole blood fraction. Error bars represent mean ± standard deviation.

## Claims

1. A self-microemulsifying drug delivery system (SMEDDS) comprising CRV431: 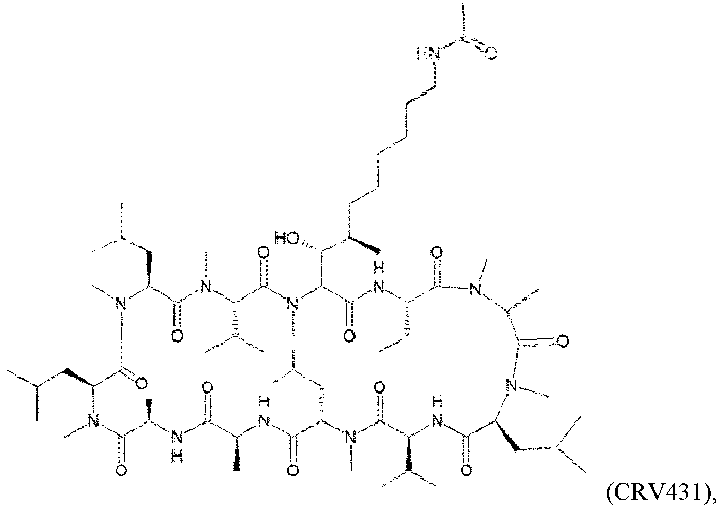 or a pharmaceutically acceptable salt thereof, the SMEDDS further comprising Vitamin E, Maisine^{®} CC, propylene glycol, Transcutol^{®}, ethanol, and Cremophor^{®} RH40 at a weight ratio, respectively, of about (0.75-1.5)/(0.5-2)/(2-5)/(2-5)/(2-2.4)/(4-8).

2. The self-microemulsifying drug delivery system of claim 1, wherein the system comprises CRV431 at a concentration from about 10 mg/mL to about 90 mg/mL.

3. A pharmaceutical composition comprising:
(a) CRV431 at a concentration of from about 10 mg/mL to about 90 mg/mL: 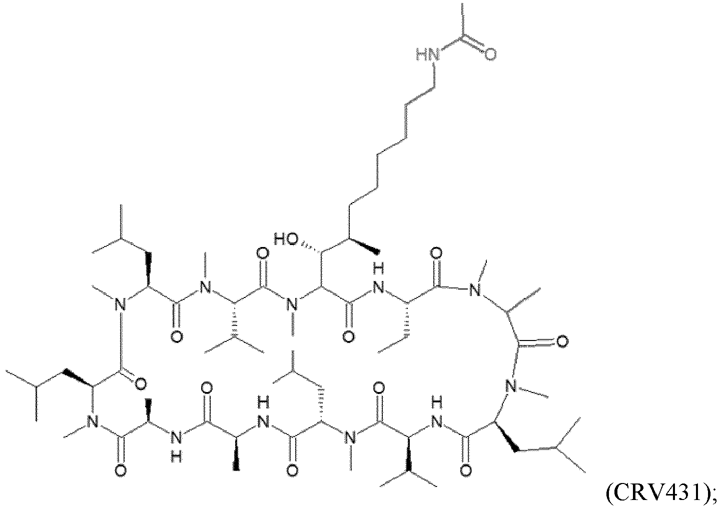
(b) Vitamin E;
(c) Maisine^{®} CC;
(d) propylene glycol;
(e) Transcutol^{®};
(f) ethanol; and
(g) Cremophor^{®} RH40, wherein Vitamin E, Maisine^{®} CC, propylene glycol, Transcutol^{®}, ethanol, and Cremophor^{®} RH40 are at a weight ratio, respectively, of about (0.75-1.5)/(0.5-2)/(2-5)/(2-5)/(2-2.4)/(4-8).

4. A self-microemulsifying drug delivery system (SMEDDS) of any one of claims 1-2 or a pharmaceutical composition of claim 3 for use as a medicament or for use in the treatment or prevention of a disease in a subject.

5. The SMEDDS or the pharmaceutical composition for use of claim 4, for use in the treatment or prevention of a disease in a subject, wherein the disease is a severe liver disease, hepatitis B (HBV), liver fibrosis, or hepatocellular carcinoma.

6. The SMEDDS or the pharmaceutical composition for use of any one of claims 4-5, wherein an area under curve (AUC) of a plot of a concentration of CRV431 in a blood of the subject over time is from about 5000 ng.hr/ml to about 150000 ng.hr/ml.

7. The SMEDDS or the pharmaceutical composition for use of any one of claims 4-6, wherein a maximum concentration (Cₘₐₓ) of CRV431 in a blood of the subject is from about 1500 ng/ml to about 2500 ng/ml, and/or wherein a time (Tₘₐₓ) to reach the maximum concentration of CRV431 in a blood of the subject is from about 0.5 hour to about 8 hours.

8. The SMEDDS or the pharmaceutical composition for use of any one of claims 4-7, wherein an elimination half-life (T_{1/2}) of CRV431 in a blood of the subject is from about 10 hours to about 200 hours.

9. The SMEDDS or the pharmaceutical composition for use of any one of claims 4-8, wherein a concentration of CRV431 in a liver of the subject relative to a concentration of CRV431 in a blood of the subject is from about 1 to about 20.

10. The SMEDDS or the pharmaceutical composition for use of any one of claims 4-9, wherein a therapeutically effective amount of the SMEDDS, the pharmaceutical composition, or CRV431 is from about 0.5 mg/kg to about 5 mg/kg.

11. The SMEDDS or the pharmaceutical composition for use of any one of claims 4-10, thereby a symptom of the disease in the subject is alleviated and/or a severity of the disease in the subject decreases, thereby a function of a liver of the subject is improved, and/or thereby a load of a virus causing the disease decreases.

12. The SMEDDS, the pharmaceutical composition or the SMEDDS or pharmaceutical composition for use of any of the preceding claims, wherein the pharmaceutical composition or the SMEDDS is stable at room temperature, and/or wherein the pharmaceutical composition or the SMEDDS is stable for from at least about 25 days to at least about 200 days.

13. The SMEDDS, the pharmaceutical composition or the SMEDDS or pharmaceutical composition for use of any of the preceding claims, wherein the diameter of particles formed by the pharmaceutical composition or the SMEDDS dispersed in an aqueous solution is from about 15 nm to about 40 nm.

14. The SMEDDS, the pharmaceutical composition or the SMEDDS or pharmaceutical composition for use of any of the preceding claims, wherein the concentration of CRV431 is from about 50 mg/mL to about 90 mg/mL, optionally wherein the concentration of CRV431 is about 70 mg/mL.

15. The SMEDDS, the pharmaceutical composition or the SMEDDS or pharmaceutical composition for use of any of the preceding claims, wherein Vitamin E, Maisine^{®} CC, propylene glycol, Transcutol^{®}, ethanol, and Cremophor^{®} RH40 are at a weight ratio, respectively, of about 1/1/5/5/2.4/4 to about 1/1.5/2.5/5/2.4/5.

16. The SMEDDS, the pharmaceutical composition or the SMEDDS or pharmaceutical composition for use of any of the preceding claims, wherein Vitamin E, Maisine^{®} CC, propylene glycol, Transcutol^{®}, ethanol, and Cremophor^{®} RH40 are at a weight ratio, respectively, of about 1/1.5/2.5/5/2.4/5.

## Patentansprüche

1. Selbstmikroemulgierendes Arzneimittelabgabesystem (Self-Microemulsifying Drug Delivery System, SMEDDS), das CRV431 umfasst: 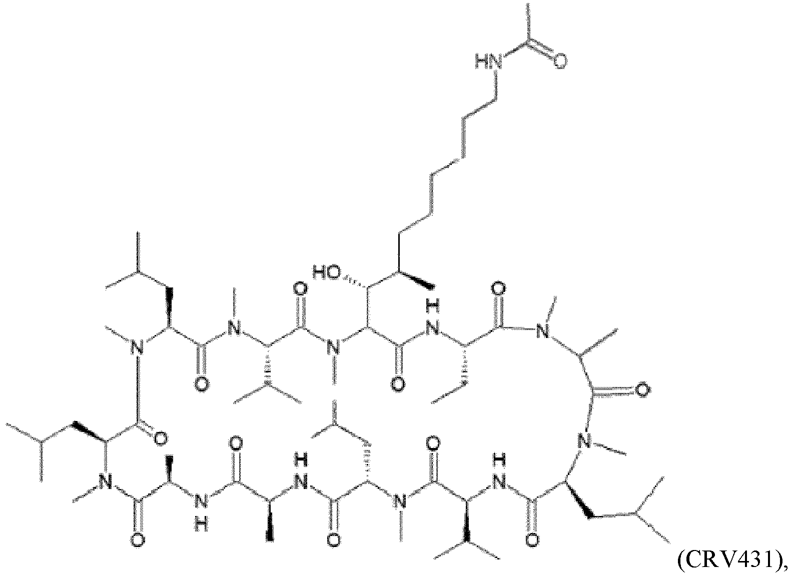 oder ein pharmazeutisch annehmbares Salz davon, wobei das SMEDDS ferner Vitamin E, Maisine^{®} CC, Propylenglykol, Transcutol^{®}, Ethanol und Cremophor^{®} RH40 in einem Gewichtsverhältnis von etwa (0,75-1,5)/(0,5-2)/(2-5)/(2-5)/(2-2,4)/(4-8) umfasst.

2. Selbstmikroemulgierendes Arzneimittelabgabesystem nach Anspruch 1, wobei das System CRV431 in einer Konzentration von etwa 10 mg/ml bis etwa 90 mg/ml umfasst.

3. Pharmazeutische Zusammensetzung, die Folgendes umfasst:
(a) CRV431 in einer Konzentration von etwa 10 mg/ml bis etwa 90 mg/ml: 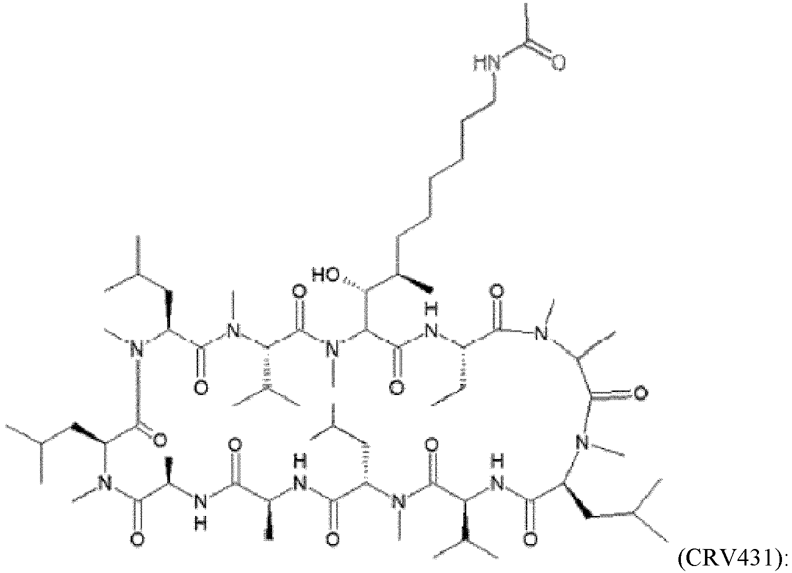
(b) Vitamin E;
(c) Maisine^{®} CC;
(d) Propylenglykol;
(e) Transcutol^{®},
(f) Ethanol; und
(g) Cremophor^{®} RH40, wobei Vitamin E, Maisine^{®} CC, Propylenglykol, Transcutol^{®}, Ethanol und Cremophor^{®} RH40 jeweils in einem Gewichtsverhältnis von etwa (0,75-1,5)/(0,5-2)/(2-5)/(2-5)/(2-2,4)/(4-8) stehen.

4. Selbstmikroemulgierendes Arzneimittelabgabesystem (SMEDDS) nach einem der Ansprüche 1 bis 2 oder pharmazeutische Zusammensetzung nach Anspruch 3 zur Verwendung als Arzneimittel oder zur Verwendung bei der Behandlung oder Vorbeugung einer Krankheit bei einem Patienten.

5. SMEDDS oder die pharmazeutische Zusammensetzung zur Verwendung nach Anspruch 4, zur Verwendung bei der Behandlung oder Vorbeugung einer Krankheit in einem Patienten, wobei die Krankheit eine schwere Lebererkrankung, Hepatitis B (HBV), Leberfibrose oder ein hepatozelluläres Karzinom ist.

6. SMEDDS oder pharmazeutische Zusammensetzung zur Verwendung nach einem der Ansprüche 4 bis 5, wobei eine Fläche unter der Kurve (Area Under Curve, AUC) eines Diagramms einer Konzentration von CRV431 im Blut des Patienten über die Zeit von etwa 5000 ng.hr/ml bis etwa 150000 ng.hr/ml beträgt.

7. SMEDDS oder pharmazeutische Zusammensetzung zur Verwendung nach einem der Ansprüche 4 bis 6, wobei eine maximale Konzentration (Cₘₐₓ) von CRV431 im Blut des Patienten von etwa 1500 ng/ml bis etwa 2500 ng/ml beträgt, und/oder wobei die Zeit (Tₘₐₓ) bis zum Erreichen der maximalen Konzentration von CRV431 im Blut des Patienten von etwa 0,5 Stunden bis etwa 8 Stunden beträgt.

8. SMEDDS oder pharmazeutische Zusammensetzung zur Verwendung nach einem der Ansprüche 4 bis 7, wobei die Eliminationshalbwertszeit (T_{1/2}) von CRV431 im Blut des Patienten etwa 10 Stunden bis etwa 200 Stunden beträgt.

9. SMEDDS oder pharmazeutische Zusammensetzung zur Verwendung nach einem der Ansprüche 4 bis 8, wobei die Konzentration von CRV431 in der Leber des Patienten im Verhältnis zur Konzentration von CRV431 im Blut des Patienten etwa 1 bis etwa 20 beträgt.

10. SMEDDS oder pharmazeutische Zusammensetzung zur Verwendung nach einem der Ansprüche 4 bis 9, wobei eine therapeutisch wirksame Menge des SMEDDS, der pharmazeutischen Zusammensetzung oder von CRV431 etwa 0,5 mg/kg bis etwa 5 mg/kg beträgt.

11. SMEDDS oder pharmazeutische Zusammensetzung zur Verwendung nach einem der Ansprüche 4 bis 10, wodurch ein Symptom der Krankheit in dem Patienten gelindert wird und/oder ein Schweregrad der Krankheit in dem Patienten abnimmt, wodurch eine Funktion einer Leber des Patienten verbessert wird und/oder wodurch eine Last eines die Krankheit verursachenden Virus abnimmt.

12. SMEDDS, pharmazeutische Zusammensetzung oder SMEDDS oder pharmazeutische Zusammensetzung zur Verwendung nach einem der vorhergehenden Ansprüche, wobei die pharmazeutische Zusammensetzung oder das SMEDDS bei Raumtemperatur stabil ist, und/oder wobei die pharmazeutische Zusammensetzung oder das SMEDDS für mindestens etwa 25 Tage bis mindestens etwa 200 Tage stabil ist.

13. SMEDDS, pharmazeutische Zusammensetzung oder SMEDDS oder pharmazeutische Zusammensetzung zur Verwendung nach einem der vorhergehenden Ansprüche, wobei der Durchmesser der durch die pharmazeutische Zusammensetzung oder das SMEDDS gebildeten Partikel, die in einer wässrigen Lösung dispergiert sind, von etwa 15 nm bis etwa 40 nm beträgt.

14. SMEDDS, pharmazeutische Zusammensetzung oder SMEDDS oder pharmazeutische Zusammensetzung zur Verwendung nach einem der vorhergehenden Ansprüche, wobei die Konzentration von CRV431 von etwa 50 mg/ml bis etwa 90 mg/ml beträgt, optionalwobei die Konzentration von CRV431 etwa 70 mg/ml beträgt.

15. SMEDDS, pharmazeutische Zusammensetzung oder SMEDDS oder pharmazeutische Zusammensetzung zur Verwendung nach einem der vorhergehenden Ansprüche, wobei Vitamin E, Maisine^{®} CC,Propylenglykol, Transcutol^{®}, Ethanol und Cremophor^{®} RH40 jeweils in einem Gewichtsverhältnis von etwa 1/1/5/5/2,4/4 bis etwa 1/1,5/2,5/5/2,4/5 stehen.

16. SMEDDS, pharmazeutische Zusammensetzung oder SMEDDS oder pharmazeutische Zusammensetzung zur Verwendung nach einem der vorhergehenden Ansprüche, wobei Vitamin E, Maisine^{®} CC, Propylenglykol, Transcutol^{®}, Ethanol und Cremophor^{®} RH40 jeweils in einem Gewichtsverhältnis von etwa 1/1,5/2,5/5/2,4/5 stehen.

## Revendications

1. Système d'administration de médicament auto-microémulsifiant (SMEDDS) comprenant CRV431 : 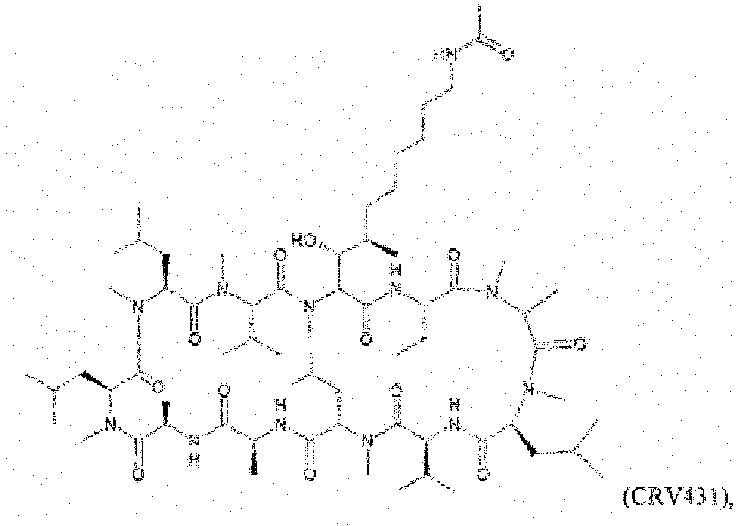 ou un sel pharmaceutiquement acceptable de celui-ci, le SMEDDS comprenant en outre vitamine E, Maisine^{®} CC, propylène glycol, Transcutol^{®}, éthanol et Cremophor^{®} RH40 dans un rapport pondéral, respectivement, d'environ (0,75-1,5)/(0,5-2)/(2-5)/(2-5)/(2-2,4)/(4-8).

2. Système d'administration de médicament auto-microémulsifiant selon la revendication 1, dans lequel le système comprend CRV431 à une concentration d'environ 10 mg/ml à environ 90 mg/ml.

3. Composition pharmaceutique comprenant :
(a) CRV431 à une concentration d'environ 10 mg/ml à environ 90 mg/ml : 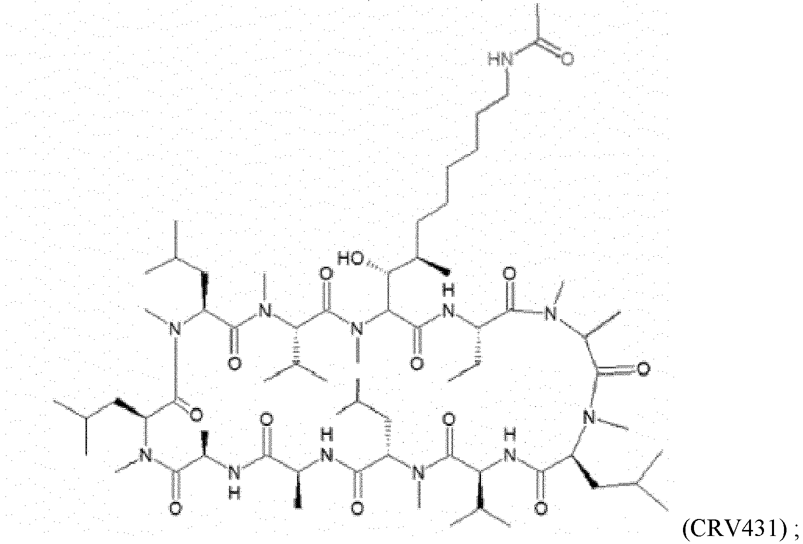
(b) vitamine E ;
(c) Maisine^{®} CC ;
(d) propylène glycol ;
(e) Transcutol^{®} ;
(f) éthanol ; et
(g) Cremophor^{®} RH40, dans lequel vitamine E, Maisine^{®} CC, propylène glycol, Transcutol^{®}, éthanol et Cremophor^{®} RH40 sont à un rapport pondéral, respectivement, d'environ (0,75-1,5)/(0,5-2)/ (2-5 )/(2-5)/(2-2,4)/(4-8).

4. Système d'administration de médicament auto-microémulsifiant (SMEDDS) selon l'une quelconque des revendications 1 à 2 ou composition pharmaceutique selon la revendication 3 pour une utilisation comme médicament ou pour une utilisation dans le traitement ou la prévention d'une maladie chez un sujet.

5. SMEDDS ou composition pharmaceutique pour une utilisation selon la revendication 4, pour une utilisation dans le traitement ou la prévention d'une maladie chez un sujet, dans laquelle la maladie est une maladie hépatique grave, l'hépatite B (VHB), une fibrose hépatique ou un carcinome hépatocellulaire.

6. SMEDDS ou composition pharmaceutique pour une utilisation selon l'une quelconque des revendications 4 à 5, dans lequel une aire sous la courbe (AUC) d'un tracé d'une concentration de CRV431 dans le sang du sujet au fil du temps est d'environ 5000 ng.h/ml à environ 150 000 ng.h/ml.

7. SMEDDS ou composition pharmaceutique pour une utilisation selon l'une quelconque des revendications 4 à 6, dans lequel une concentration maximale (Cₘₐₓ) de CRV431 dans le sang du sujet est d'environ 1500 ng/ml à environ 2500 ng/ml, et/ou dans lequel un temps (Tₘₐₓ) pour atteindre la concentration maximale de CRV431 dans le sang du sujet est d'environ 0,5 heure à environ 8 heures.

8. SMEDDS ou composition pharmaceutique pour une utilisation selon l'une quelconque des revendications 4 à 7, dans lequel une demi-vie d'élimination (T_{1/2}) de CRV431 dans le sang du sujet est d'environ 10 heures à environ 200 heures.

9. SMEDDS ou composition pharmaceutique pour une utilisation selon l'une quelconque des revendications 4 à 8, dans lequel une concentration de CRV431 dans le foie du sujet par rapport à une concentration de CRV431 dans le sang du sujet est d'environ 1 à environ 20.

10. SMEDDS ou composition pharmaceutique pour une utilisation selon l'une quelconque des revendications 4 à 9, dans lequel une quantité thérapeutiquement efficace du SMEDDS, de la composition pharmaceutique ou du CRV431 est d'environ 0,5 mg/kg à environ 5 mg/kg.

11. SMEDDS ou composition pharmaceutique pour une utilisation selon l'une quelconque des revendications 4 à 10, ainsi un symptôme de la maladie chez le sujet est atténué et/ou une sévérité de la maladie chez le sujet diminue, ainsi une fonction du foie du sujet est améliorée, et/ou ainsi une charge d'un virus provoquant la maladie diminue.

12. SMEDDS, composition pharmaceutique ou SMEDDS ou composition pharmaceutique pour une utilisation selon l'une quelconque des revendications précédentes, dans lequel la composition pharmaceutique ou le SMEDDS est stable à température ambiante, et/ou dans lequel la composition pharmaceutique ou le SMEDDS est stable pendant au moins environ 25 jours à au moins environ 200 jours.

13. SMEDDS, composition pharmaceutique ou SMEDDS ou composition pharmaceutique pour une utilisation selon l'une quelconque des revendications précédentes, dans lequel le diamètre des particules formées par la composition pharmaceutique ou le SMEDDS dispersé dans une solution aqueuse est d'environ 15 nm à environ 40 nm..

14. SMEDDS, composition pharmaceutique ou SMEDDS ou composition pharmaceutique pour une utilisation selon l'une quelconque des revendications précédentes, dans lequel la concentration de CRV431 est d'environ 50 mg/ml à environ 90 mg/ml, éventuellement dans lequel la concentration de CRV431 est d'environ 70 mg/ml.

15. SMEDDS, composition pharmaceutique ou SMEDDS ou composition pharmaceutique pour une utilisation selon l'une quelconque des revendications précédentes, dans lequel vitamine E, Maisine^{®} CC, propylène glycol, Transcutol^{®}, éthanol et Cremophor^{®} RH40 sont à un rapport pondéral, respectivement, d'environ 1/1/5/5/2,4/4 à environ 1/1,5/2,5/5/2,4/5.

16. SMEDDS, composition pharmaceutique ou SMEDDS ou composition pharmaceutique pour une utilisation selon l'une quelconque des revendications précédentes, dans lequel vitamine E, Maisine^{®} CC, propylène glycol, Transcutol^{®}, éthanol et Cremophor^{®} RH40 sont à un rapport pondéral, respectivement, d'environ 1/1,5/2,5/5/2,4/5.
